# EUROPÄISCHE PATENTSCHRIFT

(11) **EP 3 344 434 B1**
(45) Veröffentlichungstag und Bekanntmachung des Hinweises auf die Patenterteilung: **16.02.2022**
(21) Anmeldenummer: 16759761.6
(22) Anmeldetag: 31.08.2016
(51) Int. Cl.: B29C 48/154, B29C 48/25, B29C 48/34, B29C 48/03, B29C 48/156

(54) **VORRICHTUNG ZUM EXTRUDIEREN EINES STRUKTURIERTEN EXTRUDATS**
APPARATUS FOR EXTRUDING A STRUCTURED EXTRUDATE
DISPOSITIF POUR L'EXTRUSION D'UN EXTRUDAT STRUCTURÉ

(30) Priorität: 31.08.2015 DE 102015114488
(43) Veröffentlichungstag der Anmeldung: 11.07.2018
(73) Patentinhaber: MaRVis Interventional GmbH, 50226 Frechen (DE)
(72) Erfinder: LUTTMANN, Arelí Graciela, 27283 Verden (DE); DLAIKAN-CAMPOS, Nasib, 52146 Würselen (DE); DÜRING, Klaus, 50226 Frechen (DE)
(74) Vertreter: HGF
(86) Internationale Anmeldenummer: PCT/EP2016/070543
(87) Internationale Veröffentlichungsnummer: WO 2017/037130

(56) Entgegenhaltungen:
- EP-A1- 2 367 177
- EP-A1- 2 444 227
- WO-A2-02/20898
- WO-A2-2015/161931
- JP-A- 2000 326 384
- US-A- 5 215 698

## Beschreibung

Die vorliegende Erfindung betrifft eine Vorrichtung und ein Verfahren zum Extrudieren eines strukturierten

### Extrudats.

Es sind Vorrichtungen zum Extrudieren von Kabeln bekannt. In der Kabelextrusion wird in einer Extrusionsvorrichtung ein Draht einer Pinole zugeführt, wobei in der Extrusionsvorrichtung ein Polymer zur Kabelummantelung auf den Draht aufgebracht wird. Auf diese Weise wird ein kunststoffummantelter Draht hergestellt.

Weiterhin sind in der Elektrotechnik Klingeldrähte und Telefonkabel bekannt. Bei deren Herstellung wird zunächst ein einzelner Draht kunststoffummantelt. Zur Herstellung von mehradrigen Kabeln werden mehrere solcher kunststoffummantelter Drähte von einem Materialbaum ausgehend zusammengefasst und anschließend durch eine Flechtmaschine hindurchgeführt, wo sie mit einem Fadengeflecht ummantelt werden. Diese Drahtgebinde werden dann in eine Extrusionsvorrichtung eingeführt. Hierbei wird das Drahtgebinde direkt in die Pinole eingeführt und dann kunststoffummantelt.

Zum Ummanteln von elektrischen Kabeln sind Extrusionseinrichtungen bekannt (z.B.: EP 2 367 177 A1, EP 0 409 011 A1), die mit einer Pinole die einzelnen Adern des Kabels einem Extrusionsraum zuführen.

Eine ähnliche Vorrichtung ist aus der EP 1 757 428 A1 zum Ummanteln eines Katheters bzw. aus der US 5,451,355 zum Ummanteln eines aus Fasern geflochtenen Rohres bekannt.

Aus der WO 02/20898 A2 geht eine ähnliche Extrusionsvorrichtung hervor, bei der die Pinole einen mit Polymerschmelze gefüllten Hohlraum aufweist.

Aus der DE 10 2008 035 573 A1 geht ein Extrusionswerkzeug hervor, mit dem Schnüre oder Drähte zunächst mit einem ersten Polymer und dann mit einem zweiten Polymer ummantelt werden, wobei dies in einem einzigen Werkzeug erfolgt.

In der US 5,215,698 ist eine Vorrichtung zur Herstellung von Kabeln offenbart. Diese Vorrichtung umfasst einen Extrusionskopf mit einer Durchgangsöffnung, über die Drähte einem Extrusionsraum zuführbar sind. Die Durchgangsöffnung weist einen zylindrischen und einen sich konisch verjüngenden Abschnitt auf.

Aus der WO 2007/000148 A2 geht ein stabförmiger Körper hervor, der zur Ausbildung medizinischer Instrumente, wie zum Beispiel Katheter oder Führungsdrähte für Katheter, vorgesehen ist. Dieser stabförmige Körper besteht aus einem oder mehreren Filamenten und einem nicht-ferromagnetischen Matrixwerkstoff, wobei der Matrixwerkstoff die Filamente umschließt. In den Matrixwerkstoff ist eine Dotierung aus magnetresonanztomographische Artefakte erzeugenden Partikeln eingebracht.

In der WO 2009/141165 A2 ist ein medizinisches Instrument offenbart, das in einen menschlichen oder tierischen Körper einführbar ist, wobei das medizinische Instrument einen Instrumentenkörper aufweist. Der Instrumentenkörper weist zumindest einen schlecht elektrisch leitenden stabförmigen Körper auf, der aus einem Matrixwerkstoff und nicht-metallischen Filamenten ausgebildet ist. Dieses medizinische Instrument zeichnet sich dadurch aus, dass der stabförmige Körper mit einem Röntgenmarker dotiert ist, und das medizinische Instrument einen MR-Marker aufweist.

Aus der WO 2012/052159 A2 geht ein stabförmiger Körper und ein medizinisches Instrument hervor. Der stabförmige Körper umfasst ein oder mehrere nicht-metallische Filamente und ein nicht-ferromagnetisches Matrixmaterial. Das Matrixmaterial umschließt und/oder verklebt die Filamente. Markerpartikel zum Erzeugen eines Signals in der Magnetresonanz- oder in der Röntgenbildgebung sind in den stabförmigen Körper eingebracht.

In der WO 2013/072067 A1 ist eine Vorrichtung zum Extrudieren eines medizinischen Instruments, das in einen menschlichen oder tierischen Körper einführbar ist, offenbart. Die Vorrichtung umfasst eine Einrichtung zum Zuführen stabförmiger Körper, eine Extrusionseinrichtung mit einem Gehäuse, wobei das Gehäuse eine umlaufende Seitenwandung aufweist, die an einem in Produktionsrichtung vorne liegenden Ende mit einer eine Austrittsdüse aufweisenden Düsenwandung und am in Produktionsrichtung hinten liegenden Ende mit einer Pinole versehen ist. Der Raum im Gehäuse zwischen der Pinole, der Seitenwandung und der Austrittsdüse begrenzt einen Extrusionsraum, wobei das Gehäuse im Bereich des Extrusionsraums mit einer Polymerzuführeinrichtung versehen ist. Weiterhin ist eine sich in Produktionsrichtung erstreckende Kanüleneinrichtung vorgesehen, die ausgebildet ist, um zumindest einen stabförmigen Körper von der Einrichtung zum Zuführen stabförmiger Körper bis in den Extrusionsraum in einer vorbestimmten räumlichen Anordnung einzubringen, die zumindest eine rohrförmige Kanüle mit einem in Produktionsrichtung hinten liegenden Zuführende und einem in Produktionsrichtung vorne liegenden Austrittsende aufweist, wobei die Kanüleneinrichtung in etwa in geradliniger Flucht zur Austrittsdüse angeordnet ist und sich durch die Pinole derart erstreckt, dass ihr in Produktionsrichtung liegendes Austrittsende beabstandet von der Austrittsdüse im Extrusionsraum endet.

In dem Fachbuch von Walter Michaeli "Extrusionswerkzeuge für Kunststoffe" (München Wien: Carl Hanser Verlag, 1991.-ISBN 3-446-15637-2) ist auf Seite 170 im Bild 5.54 ein Extrusionswerkzeug offenbart, das eine Pinole zum Führen eines Leiters aufweist, wobei die Pinole an dem zur Düse des Werkzeuges weisenden Ende den Leiter enger umschließt als im übrigen Bereich.

Die DE 10 2008 035 573 A1 offenbart ein Extrusionswerkzeug, das eine Extrusionsdüse aufweist. In einem Führungselement sind mehrere Kanäle ausgebildet, die zueinander parallel verlaufen. Durch diese Kanäle sollen beispielsweise Drähte zugeführt werden, die ummantelt werden.

Aus der DE 10 2011 118 719 A1 geht eine Extrusionsvorrichtung hervor, die in Produktionsrichtung nach einer Austrittsdüse eine Rolleneinrichtung zum Führen eines herzustellenden Instrumentes aufweist.

Aus der DE 10 2014 005 901 A1 geht eine Vorrichtung zum Extrudieren eines strukturierten Extrudats, das in einen menschlichen oder tierischen Körper einbringbar ist, hervor. Diese Vorrichtung umfasst eine Extrusionseinrichtung mit einem Gehäuse, wobei das Gehäuse eine umlaufende Seitenwandung aufweist, die an einem in Produktionsrichtung vorne liegenden Ende mit einer eine Austrittsdüse aufweisenden Düsenwandung und in Produktionsrichtung davor liegend mit einer Pinole versehen ist, wobei der Raum im Gehäuse zwischen der Pinole, der Seitenwandung und der Austrittsdüse einen Extrusionsraum begrenzt und das Gehäuse im Bereich des Extrusionsraumes mit einer Polymerzuführeinrichtung versehen ist. In der Pinole ist zumindest ein sich in Produktionsrichtung erstreckender Führungskanal ausgebildet, um zumindest einen stabförmigen Körper von der Einrichtung zum Zuführen stabförmiger Körper bis in den Extrusionsraum einbringen zu können, wobei der zumindest eine Führungskanal in etwa in geradliniger Flucht zur Austrittsdüse angeordnet ist.

In der JP 2000 326384 A ist eine Vorrichtung zum Extrudieren eines Katheters beschrieben. Ein entsprechender Katheder soll durch diskontinuierliche Beaufschlagung eines Kerndrahts mittels eines Extrudats herstellbar sein. Eine Pinole dieser Vorrichtung ist dreiteilig ausgebildet, wobei ein in den Extrusionsraum mündender und in Produktionsrichtung vorne liegender Teil der Pinole wohl einen Führungskanal für den Kerndraht aufweist, der eine im Wesentlichen konstante Konizität besitzt.

In der WO 02/20898 A2 ist eine Düse für eine Extrusionsvorrichtung offenbart mittels der ein Bündel Stränge imprägniert werden kann und wobei die imprägnierten Stränge beschichtet werden, um aus den imprägnierten Strängen ein Verbundkabel auszubilden.

Aus der EP 2 444 227 A1 geht ein Verfahren zum kontinuierlichen Herstellen eines LED-Bandes hervor. Ein Extrusionswerkzeug weist einen oberen Schmelzekanal und einen unteren Schmelzekanal auf, welche in einen formgebenden Düsenkanal münden, durch den ein LED-Band hindurchgeführt werden kann. In dem formgebenden Düsenkanal erhält das LED-Band seine endgültige Form. Dem Düsenkanal ist ein sich entgegen der Transportrichtung aufweitender Zuführkanal vorgeordnet.

Aufgabe der vorliegenden Erfindung ist es, eine Vorrichtung und ein Verfahren zum Extrudieren von strukturierten Extrudaten bereitzustellen, bei dessen Herstellung bestimmte mechanische Eigenschaften erfüllt werden können. Insbesondere soll es möglich sein, einzelne stabförmige Körper in einer vorbestimmten Position im strukturierten Extrudats anzuordnen.

Eine weitere Aufgabe der vorliegenden Erfindung ist es, eine gegenüber den in der WO 2013/072067 A1 und der DE 10 2014 005 901 A1 beschriebenen Vorrichtungen verbesserte Vorrichtung bereitzustellen, die insbesondere kostengünstiger herstellbar, einfacher aufgebaut ist und zudem eine bessere Reproduzierbarkeit ermöglicht.

Die Erfindung weist zur Lösung dieser Aufgabe die in den unabhängigen Patentansprüchen angegebenen Merkmale auf. Vorteilhafte Ausgestaltungen hiervon sind in den jeweiligen Unteransprüchen angegeben.

Erfindungsgemäß ist eine Vorrichtung zum Extrudieren eines strukturierten Extrudats, vorgesehen. Diese Vorrichtung umfasst ein Gehäuse, wobei das Gehäuse eine umlaufende Seitenwandung aufweist, die an einem in Produktionsrichtung vorne liegenden Ende mit einer eine Austrittsdüse aufweisenden Düsenwandung und am entgegen der Produktionsrichtung hinten liegenden Ende mit einer Pinole versehen ist, wobei der Raum im Gehäuse zwischen der Pinole, der Seitenwandung und der Austrittsdüse einen Extrusionsraum begrenzt und das Gehäuse im Bereich des Extrusionsraumes mit einer Polymerzuführeinrichtung verbindbar ist.

In der Pinole ist ein sich in Produktionsrichtung erstreckender Führungskanal ausgebildet, um zumindest einen stabförmigen Körper von einer Einrichtung zum Zuführen stabförmiger Körper bis in den Extrusionsraum einzubringen, wobei der Führungskanal in etwa in geradliniger Flucht zur Austrittsdüse angeordnet ist. Der Führungskanal weist über seine gesamte Länge eine konstante Konizität auf. Die Vorrichtung zeichnet sich dadurch aus, dass ein peripherer Führungskanal neben dem zentralen Führungskanal vorgesehen ist der über seine gesamte Länge eine konstante Konizität aufweist und sich in Produktionsrichtung verjüngt.

Unter einer konstanten Konizität wird im Rahmen der vorliegenden Erfindung verstanden, dass die Rotationsfläche, gebildet durch eine um eine Achse rotierende Kurve, gegenüber dieser Achse über die gesamte Länge den gleichen Winkel bezüglich der Achse aufweist bzw., dass die Steigung des kegelförmigen Zuführkanals bzw. dessen Neigungswinkel gegenüber der Produktionsrichtung konstant bleibt.

Dadurch, dass der Führungskanal über seine gesamte Länge eine konstante Konizität aufweist, sind im Führungskanal keinerlei Ecken und Kanten vorhanden. Dadurch können sich an Übergängen Kanten, Verjüngungen, Engstellen und Ecken keine Materialteilchen in schlecht zugänglichen Bereichen ablagern und den Kanal verstopfen.

Der Führungskanal erstreckt sich über die gesamte Länge der Pinole in Produktionsrichtung. Demgemäß weist der Führungskanal über die gesamte Länge der Pinole eine konstante Konizität auf.

Zudem wird das Reinigen der Führungskanäle erheblich erleichtert, da weniger Material im Führungskanal verbleibt bzw. in diesen eindringt.

Dadurch, dass der Führungskanal sich in Produktionsrichtung verjüngt, d.h. dass die in Produktionsrichtung liegende Öffnung des Führungskanals einen geringeren Durchmesser als die in Produktionsrichtung hinten liegende Öffnung des Führungskanals aufweist, ist es möglich, im strukturierten Extrudat stabförmige Körper präzise anzuordnen.

Dadurch, dass der Führungskanal über die gesamte Länge der Pinole eine konstante Konizität aufweist, ist es möglich, den oder die stabförmigen Körper im Querschnitt eines strukturierten Extrudats an vorbestimmter Position präzise anzuordnen.

Dadurch, dass die in Produktionsrichtung hinten liegende Öffnung des Führungskanals einen größeren Durchmesser aufweist, vereinfacht sich das Einfädeln von stabförmigen Körpern in den Zuführkanal erheblich.

Ein solches Einfädeln ist insbesondere beim Einfädeln mehrerer stabförmiger Körper nur dann einfach möglich, wenn die in Produktionsrichtung hinten liegende Öffnung eines Führungskanals einen ausreichend großen Durchmesser aufweist, weil ansonsten die Einführung des/der stabförmigen Körper auf engem Raum sehr gezielt erfolgen muss, was häufig eine Beschädigung der stabförmigen Körper zur Folge hat, u.a. ein Verbrennen des Matrixmaterials durch Kontakt mit der heißen Pinole.

Der vorliegenden Erfindung liegen die Erkenntnisse zugrunde, dass bei einer Vorrichtung zum Extrudieren eines strukturierten Extrudats ein anderer Aufbau der Pinole erforderlich ist als z. B. bei einer herkömmlichen Extrusionsvorrichtung zum Extrudieren bzw. Ummanteln von Kabeln, um Stromkabel auszubilden.

Beim Zuführen eines zu ummantelnden Materials mittels einer Rolleneinrichtung kommt es aufgrund des Abrollens des zu ummantelnden Materials von entsprechenden Spulen einer Spuleneinrichtung zu ungleichmäßigen Schwingungen und damit verbundenen Vibrationen. Diese sind umso größer, je mehr Raum dem Material zum Schwingen zur Verfügung steht. Dies spielt bei herkömmlichen Extrusionsvorrichtungen zum Ummanteln von Kabeln keine Rolle, ist aber bei einem Mikroextrusionsverfahren mit sehr kleinen Produktdurchmessern von sehr großer Bedeutung. Durch einen größeren freien Raum in einer Pinole einer Extrusionsvorrichtung kommt es jedoch zu größeren Schwingungen des Ausgangsmaterials. Da konstruktionsbedingt Kanten in einem zylindrischen und einem konusförmigen Abschnitt einer Pinole, insbesondere im Übergangsbereich von zylindrisch auf konisch und an dessen Öffnung im Bereich des Extrusionsraums sowie auch an einer Düsenöffnung einer solchen Extrusionsvorrichtung, vorhanden sind, würde ein stabförmiger Körper an diesen schleifen und dadurch beschädigt werden. Da es vorteilhaft ist, den Kontakt des stabförmigen Körpers mit der Wandung des Führungskanal so kurz wie möglich auszugestalten, ist die Verhinderung von Schwingungen und ein schwinungsarmes Durchführen des stabförmigen Körpers durch den Führungskanal und aus der Pinole hinaus vorteilhaft.

Mittels der erfindungsgemäßen konstanten Konizität eines oder mehrerer Führungskanäle sind nahezu keine Kanten bzw. nur "weiche" Kanten vorhanden. Weiterhin ist der Raum für Schwingungen erheblich kleiner, da bereits unmittelbar nach dem Eintritt eines stabförmigen Körpers in die Pinole diese durch den Führungskanal mit konstanter Konizität geführt wird.

Die erfindungsgemäßen stabförmigen Körper sind im Vergleich zu den aus dem Stand der Technik bekannten Kabeln hinsichtlich ihrer mechanischen Eigenschaften extrem dünn und daher empfindlich. Diese Empfindlichkeit betrifft auch das Material, aus dem die stabförmigen Körper ausgebildet sind. Die Temperatur zur Herstellung des strukturierten Extrudats, bei dem dieses extrudiert wird, liegt in etwa bei ca. 150 bis 400°C bzw. ca. 180 bis 300°C bzw. 200 bis 250°C, wobei das in dem stabförmigen Körper vorhandene Matrixmaterial eine unter der Extrusionstemperatur liegende Glastemperatur aufweisen kann, bei deren Erreichen oder sogar schon darunter thermische Schädigungen des Matrixmaterials eintreten können. Daher liegt der vorliegenden Erfindung insbesondere die Erkenntnis zugrunde, dass bei Verwendung eines stabförmigen Körpers ein Kontakt mit der Pinolenoberfläche weitestgehend vermieden werden soll und insbesondere ein Streifen entlang heißer, im Führungskanal ausgebildeter Kanten vermindert werden muss, um den stabförmigen Körper nicht zu beschädigen, wodurch dessen mechanische Steifigkeit reduziert werden würde. Außerdem kann es nach Beschädigung des Matrixmaterials zu Verstopfungen in den Führungskanälen durch freiliegende und an Kanten des Führungskanals abbrechende Glasfasern kommen, so dass sich Knäuel kurzer Glasfaserstücke ausbilden, welche sich innerhalb des Führungskanals ablagern und dann dort mit Matrixmaterial verbinden und in der Folge diesen verstopfen können. Erfahrungsgemäß erfolgt dies vor allem an Ecken, wo sich der Durchmesser des Kanals verjüngt.

Derartige Führungskanäle mit konstanter Konizität sind schwierig herzustellen. Nach zahlreichen Versuchen hat sich herausgestellt, dass derartige dünne Führungskanäle mit konstanter Konizität durch Drahterosion herstellbar sind.

Anfangs wurde versucht, mehrere Bauteile in der Pinole und auch in Produktionsrichtung vor und hinter der Pinole vorzusehen, um einen Kantenkontakt der stabförmigen Körper mit Bauteilen, insbesondere mit dem Führungskanal der Pinole, zu verhindern oder diesen zumindest zu reduzieren und auch die Schwingungen im Führungskanal zu reduzieren. Die Lösungen mit mehreren Bauteilen haben sich jedoch nicht als effizient genug erwiesen, um stabförmige Körper über einen oder mehrere Führungskanäle beschädigungsfrei dem Extrusionsraum zuzuführen und diese anschließend zu extrudieren.

Durch den Führungskanal mit konstanter Konizität wird ein exaktes lineares Ausrichten eines stabförmigen Körpers gegenüber dem Stand der Technik erheblich vereinfacht.

Mittels des Führungskanals wird ein stabförmiger Körper bis zur Umhüllung mit Polymer exakt geführt und im Raum ausgerichtet.

Es hat sich gezeigt, dass in der Pinole ausgebildete Führungskanäle gegenüber der aus dem Stand der Technik bekannten Kanülenanordnung oder der aus dem Stand der Technik bekannten Zuführeinrichtungen den erheblichen Vorteil aufweisen, dass diese durch das Fertigungsverfahren nicht in ihrer Ausrichtung in Produktionsrichtung verändert bzw. verstellt werden, sodass eine präzise Führung der stabförmigen Körper durch die Pinole in dem Extrusionsraum möglich ist. Außerdem wird das Risiko von Beschädigungen der stabförmigen Körper durch Kanten, Ecken oder Winkeländerungen beim Führen in dem Extrusionsraum sowie des Vibrierens einer Kanülenanordnung durch die in der erfindungsgemäßen Pinole ausgebildeten Führungskanäle unterbunden. Ein Kontakt des stabförmigen Körpers mit der Wandung des Führungskanals kann zu einer übermäßigen Erhitzung des stabförmigen Körpers und dessen möglicher Materialveränderung bzw. -zerstörung führen. Bei einem nicht konischen Führungskanal, der stufenweise Änderungen des Durchmessers, Kanten oder Ecken aufweist, kommt es zwangsläufig zu derartigen Kontakten an diesen Positionen.

Durch das exakte Fertigungsverfahren der erfindungsgemäßen Pinole wird die exakte geometrische Anordnung der Führungskanäle und damit der stabförmigen Körper auf einfache Weise sichergestellt und damit wird die Reproduzierbarkeit des hergestellten Extrudats erheblich verbessert.

Durch die exakte Führung im Führungskanal und einen kurzen Abstand zwischen der in Produktionsrichtung vorne liegenden Öffnung des Führungskanals und dem in Produktionsrichtung hinten liegenden Ende der Austrittsdüse wird zudem sicher und zuverlässig vermieden, dass einzelne stabförmige Körper nach der Umhüllung mit dem Polymer aus dem strukturierten Extrudat herausstehen. Diese Gefahr besteht aufgrund des geringen Außendurchmessers des strukturierten Extrudats, wenn auf eine Führung der stabförmigen Körper verzichtet oder ein großer Abstand benutzt würde oder die stabförmigen Körper durch Ecken oder Kanten im Extrusionsraum beschädigt oder zumindest in ihren Materialeigenschaften beeinträchtigt werden, da diese dann beim Extrudieren aufgrund des Druckes der Schmelze in ihrer Position verändert werden können.

Unter dem Begriff "in etwa in geradliniger Flucht" wird im Rahmen der vorliegenden Erfindung verstanden, dass der zentrale Führungskanal parallel zur Produktionsrichtung angeordnet ist. Dies bedeutet nicht, dass der zentrale Führungskanal exakt mit der Mitte der Austrittsdüse fluchtet, sondern dass dieser parallel zur Mittelachse der Austrittsdüse angeordnet ist.

Unter einem strukturierten Extrudat wird im Rahmen der vorliegenden Erfindung insbesondere ein Halbzeug zum Ausbilden eines medizinischen Instruments, das in einen menschlichen oder tierischen Körper einbringbar ist, verstanden. Ein mit der erfindungsgemäßen Vorrichtung hergestelltes strukturiertes Extrudat kann sehr dünn sein und einen Durchmesser von weniger als 1,5 mm, oder einen Durchmesser, der kleiner oder gleich 1 mm bzw. kleiner oder gleich 0,5 mm ist, aufweisen. Selbst ein sehr dünnes Extrudat kann mit einer vorbestimmten Struktur ausgebildet sein. Insbesondere können stabförmige Körper in dem strukturierten Extrudat exakt positioniert sein. Ist das strukturierte Extrudat ein Halbzeug zur Ausbildung eines medizinischen Instrumentes, dann hat die Positionierung der stabförmigen Körper jedenfalls erheblichen Einfluss auf die Biegesteifigkeit des strukturierten Extrudates bzw. auf das hieraus hergestellte medizinische Instrument. Weiterhin können die stabförmigen Körper mit Markern, insbesondere MR-Markern zur Sichtbarmachung in einem medizinischen, bildgebenden Verfahren versehen sein. Die Position der stabförmigen Körper im strukturierten Extrudat kann auch Einfluss auf die Sichtbarmachung des strukturierten Extrudates bzw. des hieraus hergestellten medizinischen Instrumentes haben. Eine exakte Positionierung der stabförmigen Körper ist deshalb von grundsätzlicher Bedeutung. Stabförmige Körper sind, wie es eingangs erläutert ist, beispielsweise aus der WO 2007/000148 A2 bzw. der WO 2012/052159 A2 bekannt. Derartige stabförmige Körper sind vor allem langgestreckt. Sie können auch als Fasern oder Fäden bezeichnet werden. Sie sind jedoch vorzugsweise Präzisionsfasern bzw. Präzisionsfäden mit im Wesentlichen konstantem Querschnitt bzw. konstanter Dicke über ihre gesamte Länge.

Mittels der zumindest zwei Führungskanäle wird eine gewünschte relative Anordnung von zumindest zwei stabförmigen Körpern in einem strukturierten Extrudat und der zwei stabförmigen Körper zueinander erzielt, wobei die stabförmigen Körper sehr eng benachbart zueinander anordbar sind.

Der Abstand zwischen dem in Produktionsrichtung liegenden Ende des Führungskanals und einer Eintrittsöffnung der Austrittsdüse beträgt ca. 4 bis 12 mm und insbesondere ca. 5 bis 7 mm.

Der periphere Führungskanal kann gegenüber der Produktionsrichtung in einem Winkel von 0° bis 30° bzw. von 2,5° bis 15° und insbesondere von 5° bis 10° geneigt sein.

Zudem können zumindest drei den zentralen Führungskanal konzentrisch umgebende periphere Führungskanäle vorgesehen sein.

Diese peripheren Führungskanäle sind vorzugsweise radial gleich beabstandet voneinander angeordnet.

Hierbei können der zentrale und zumindest zwei bzw. drei bzw. vier bzw. fünf bzw. sechs bzw. sieben bzw. acht bzw. neun bzw. zehn den zentralen Führungskanal umgebende periphere Führungskanäle vorgesehen sein.

Eine Innenfläche bzw. Wandung eines Führungskanals mit konstanter Konizität kann vorzugsweise eine Oberflächenrauheit R_{A} ≤ 2,0 µm R_{A} ≤ 1,5 µm und insbesondere von R_{A} ≤ 1,0 µm aufweisen.

Die mittlere Rauheit R_{A} bezeichnet die Unebenheit einer Oberflächenhöhe und gibt den mittleren Abstand eines Messpunktes auf der Oberfläche zur Mittellinie an. Die Mittellinie schneidet innerhalb einer Bezugsstrecke das wirkliche Profil so, dass die Summe der Profilabweichungen (bezogen auf die Mittellinie) minimal wird. Die mittlere Rauheit R_{A} entspricht also dem arithmetischen Mittel der betragsmäßigen Abweichung von der Mittellinie.

Eine in Produktionsrichtung vorne liegende Öffnung des zentralen Führungskanals weist einen Durchmesser von 0,2 mm bis 0,4 mm und insbesondere von 0,3 mm auf, wobei eine in Produktionsrichtung hinten liegende Öffnung des zentralen Führungskanals einen Durchmesser von 2,0 mm bis 4,0 mm und insbesondere von 3,0 mm aufweist.

Eine in Produktionsrichtung vorne liegende Öffnung eines peripheren Führungskanals weist einen Durchmesser von 0,1 mm bis 0,3 mm und insbesondere von 0,2 mm auf, wobei eine in Produktionsrichtung hinten liegende Öffnung eines peripheren Führungskanals einen Durchmesser von 3,0 mm bis 5,0 mm und insbesondere von 4,0 mm aufweist.

Gemäß einer weiteren Ausführungsform der Führungskanäle können diese neben einem runden Querschnitt auch einen Querschnitt mit einer anderen geometrischen Form aufweisen. Ein solcher Querschnitt wird als konturierter Querschnitt bezeichnet. Diese konturierten Querschnitte können beliebig miteinander kombiniert werden und weisen ebenfalls die vorstehend genannten Oberflächenrauheitswerte R_{A} auf.

Der konturierte Querschnitt des bzw. der Führungskanäle kann z. B. oval bzw. tropfenförmig, elliptisch, trapezförmig, drei- oder vier- oder vieleckig oder dergleichen ausgebildet sein. Bei jeder dieser geometrischen Formen des konturierten Querschnitts ist es möglich, dass die Kanten bezüglich einer Ebene quer zur Produktionsrichtung gerade oder konvex oder konkav ausgebildet sind. Weiterhin ist es auch möglich, dass der zentrale Führungskanal einen anderen geometrischen Querschnitt aufweist, wie der oder die peripheren Führungskanäle. Die peripheren Führungskanäle sind vorzugsweise radial gleich beabstandet voneinander angeordnet und können auch alternierend unterschiedliche konturierte Querschnitte aufweisen.

Die Form des Querschnitts der zugeführten stabförmigen Körper entspricht im Wesentlichen dem Querschnitt der Führungskanäle.

Bei einem elliptischen Querschnitt des Führungskanals ist vorgesehen, dass die zwei Punkte der Ellipse mit dem geringsten Abstand bezüglich des Schwerpunkts der Ellipse in einer zur Produktionsrichtung vertikalen Ebene radial fluchtend angeordnet sind.

Bei einem trapezförmigen Querschnitt ist vorgesehen, dass die kürzere zweier parallel zueinander verlaufender Kanten des Trapezes näher zur Produktionsrichtung angeordnet ist als die längere Kante.

Mittels derartiger Führungskanäle ist es möglich, einen größeren Anteil an stabförmigen Körpern im medizinischen Produkt anzuordnen und auf diese Weise eine höhere mechanische Steifigkeit zu erzielen, wodurch z.B. ein Abknicken eines medizinischen Instruments effektiv verhindert werden kann.

Ein mittels eines oder mehrerer Führungskanäle mit konturiertem Querschnitt hergestelltes medizinisches Instrument weist eine entsprechende Anordnung der stabförmigen Körper im strukturierten Extrudat auf, da, wie vorstehend erläutert, diese mit einem dem oder den Querschnitten der Führungskanäle im Querschnitt entsprechenden stabförmigen Körper verwendet werden.

Durch die an die geometrische Form der stabförmigen Körper angepasste geometrische Form der Führungskanäle in der Pinole wird es möglich, die in den medizinischen Instrumenten erforderliche geometrische Anordnung der stabförmigen Körper präzise zu erreichen, ohne dass es zu einem Verkanten der stabförmigen Körper in runden Führungskanälen während des Produktionsprozesses kommt. Die präzise geometrische Anordnung der stabförmigen Körper ist für die mechanische Qualität eines medizinischen Instruments von großer Bedeutung, da eine inhomogene Produktqualität mechanische Schwachstellen in das medizinische Instrument einbringt, die z.B. zu leichtem Abknicken führen können.

Vorzugsweise ist vorgesehen, dass die Anzahl der Führungskanäle der Pinole der Anzahl der stabförmigen Körper entspricht, die im strukturierten Extrudat vorgesehen werden sollen bzw. diese um 1 übersteigt.

Gemäß einem weiteren Aspekt der vorliegenden Erfindung kann die Düsenwandung im Bereich des Extrusionsraumes als ein sich in Produktionsrichtung konisch verjüngender Kegelstumpf ausgebildet sein, wobei der Kegelstumpf eine bezüglich des Extrusionsraumes konvex geformte Mantelwandung aufweist.

Dadurch, dass der Kegelstumpf eine bezüglich des Extrusionsraumes konvex geformte Mantelwandung aufweist, sind an einem Übergangsbereich von der Düsenwandung auf die Austrittsdüse keine Kanten mehr vorhanden. Ein derart abgerundeter bzw. sanfter Übergang von der Düsenwandung in einen Schmelzekanal der Austrittsdüse stellt sicher, dass im Extrusionsraum in dem Bereich, wo das strukturierte Extrudat geführt wird keine Kanten vorhanden sind, an denen die stabförmigen Körper entlang streifen. Durch das Entlangstreifen an Kanten wird das Polymer von den stabförmigen Körpern wieder abgestreift und es entsteht eine raue Oberfläche an den Stellen, wo die stabförmigen Körper an der Oberfläche liegen und nicht von Polymer überdeckt sind. Die konvex geformte Mantelwandung ermöglicht so eine hohe Oberflächenqualität des strukturierten Extrudats.

Die bezüglich des Extrusionsraumes konvex geformte Düsenwandung ist insbesondere von Bedeutung, wenn der Abstand der peripheren stabförmigen Körper im Extrudat geringer sein soll als der Abstand der peripheren Führungskanäle in der Pinole, da dann die stabförmigen Körper im Schmelzekanal näher aneinander gedrückt werden.

Gemäß einem weiteren Aspekt der vorliegenden Erfindung umfasst die Austrittsdüse einen Schmelzekanal, wobei der Schmelzekanal sich in Produktionsrichtung konisch verjüngend als ein ringförmiger Kegelstumpf mit einer Anordnungswandung ausgebildet ist.

Eine entgegen der Produktionsrichtung hinten liegende Öffnung des Schmelzekanals wird als Eintrittsöffnung bezeichnet. Eine in Produktionsrichtung vorne liegende Öffnung des Schmelzekanals wird als Austrittsöffnung bezeichnet.

Eine Innenfläche bzw. -wandung des Schmelzekanals und/oder der den Extrusionsraum begrenzenden Düsenwandung können vorzugsweise eine Oberflächenrauheit R_{A} ≤ 2,0 µm R_{A} ≤ 1,5 µm und insbesondere von R_{A} ≤ 1,0 µm aufweisen.

Dies ermöglicht einen sanfteren Übergang der stabförmigen Körper bzw. des Extrudats vom Extrusionsraum bzw. der Düsenwandung in den Schmelzekanal und gewährleistet eine glattere Oberfläche des Extrudats.

Die Eintrittsöffnung kann im Querschnitt vorzugsweise gewellt bzw. blumenförmig oder auch gezackt ausgebildet sein. Die sich daraus ergebenden, sich in Produktionsrichtung erstreckenden Konturen werden als Rinnen bzw. Ausbuchtungen bezeichnet. Die Bereiche zwischen den Ausbuchtungen werden als Einbuchtungen bezeichnet.

Die Austrittsöffnung kann im Querschnitt insbesondere kreisförmig ausgebildet sein.

Demgemäß sind in der Anordnungswandung des Schmelzekanals vom gewellten Querschnitt der Eintrittsöffnung aus sich in Produktionsrichtung erstreckende Rinnen ausgebildet, wobei die

Rinnen dann in den kreisförmigen Querschnitt der Austrittsöffnung übergehen. Daher wird die Austrittsdüse als strukturierte Austrittsdüse bezeichnet.

Die Anzahl der konturierten Rinnen kann vorzugsweise der Anzahl der peripheren Führungskanäle entsprechen.

Vorzugsweise können die Rinnen um den halben Abstandswinkel der peripheren Führungskanäle versetzt zu diesen angeordnet sein.

Das bedeutet, dass die peripheren Führungskanäle der Pinole in etwa axial fluchtend zu den Einbuchtungen des Schmelzekanals angeordnet sind. Eine derartige Ausrichtung der peripheren Führungskanäle zu den Einbuchtungen des Schmelzekanals bewirkt, dass das in den Einbuchtungen strömende Polymer im Schmelzekanal beim Übergang von dem konturierten Abschnitt hin zur runden Austrittsöffnung in den Bereich gedrückt wird, in dem die stabförmigen Körper angeordnet sind, sodass diese beim Austritt aus der Vorrichtung ausreichend mit Polymer überdeckt sind und ein rundes Extrudat bereitstellbar ist.

Weiterhin wird durch diesen Versatz ein geringfügiger Druck auf die stabförmigen Körper ausgeübt, so dass diese die exakte geometrische Anordnung im strukturierten Extrudat einnehmen können und nicht nach außen an die Oberfläche des Extrudats gedrückt werden.

Insbesondere durch die Ausbildung der Düsenwandung in Kombination mit der Ausbildung der Anordnungswandung des Schmelzekanals (strukturierte Austrittsdüse) ist es möglich das Schwingen des Extrudats zu minimieren, Riefen durch Kontakt mit Wandungen der Vorrichtung zu reduzieren und auf diese Weise ein Extrudat mit darin exakt angeordneten stabförmigen Körpern und einer glatten Oberfläche bereitzustellen.

Die endgültige äußere Geometrie des strukturierten Extrudats wird durch die Form und die Abmessung des Querschnitts des Schmelzekanals der strukturierten Austrittsdüse beeinflusst. Durch deren Ausbildung wird die exakte geometrische Anordnung der stabförmigen Körper auf einfache Weise sichergestellt und damit wird die Reproduzierbarkeit des hergestellten Extrudats erheblich verbessert. Insbesondere der Versatz der Rinnen trägt dazu bei, dass die stabförmigen Körper mit dem Polymer vollständig ummantelt sind und nicht aus diesem herausstehen.

Durch die exakte Führung im Führungskanal und einen kurzen Abstand zwischen der in Produktionsrichtung vorne liegenden Öffnung des Führungskanals und der in Produktionsrichtung hinten liegenden Eintrittsöffnung der Austrittsdüse sowie die geometrische Ausbildung des Schmelzekanals wird zudem sicher und zuverlässig vermieden, dass einzelne stabförmige Körper nach der Umhüllung mit dem Polymer aus dem strukturierten Extrudat herausstehen. Diese Gefahr besteht aufgrund des geringen Außendurchmessers des strukturierten Extrudats, wenn auf eine Führung der stabförmigen Körper verzichtet oder ein großer Abstand benutzt würde oder die stabförmigen Körper durch Ecken oder Kanten im Extrusionsraum beschädigt oder zumindest in ihren Materialeigenschaften beeinträchtigt werden, da diese dann beim Extrudieren aufgrund des Druckes der Schmelze in ihrer Position verändert werden können.

Ein erfindungsgemäßes System zum Extrudieren eines strukturierten Extrudats umfasst die vorstehend beschriebene Vorrichtung sowie eine Einrichtung zum Zuführen stabförmiger Körper, eine mit dem Extrusionsraum verbundene Polymerzuführeinrichtung, eine Kühleinrichtung und eine Abzugs- und/oder Führungseinrichtung.

In Produktionsrichtung ist nach der Austrittsdüse die Kühleinrichtung, insbesondere ein Wasserbad zum Kühlen des strukturierten Extrudats angeordnet. Im Wasserbad erfolgt eine Aushärtung des den oder die stabförmigen Körper einbettenden und/oder umgebenden Polymers. Nach dem Wasserbad kann in Produktionsrichtung eine Abzugseinrichtung angeordnet sein.

Zwischen dem Wasserbad und der Abzugseinrichtung kann eine Ausrichteinrichtung vorgesehen werden, die die Einstellung der Anordnung der stabförmigen Körper im strukturierten Extrudat unterstützt. Diese kann z.B. als schwammartiger Körper mit Einschnitt ausgebildet sein kann, wobei durch den Einschnitt das strukturierte Extrudat gezogen und geführt wird.

Die Ausrichteinrichtung kann als Fixierplatte mit einer Ausrichtbohrung ausgebildet sein, die in geradliniger axialer Flucht zum Schmelzekanal der Austrittsdüse angeordnet ist. Bevorzugt ist eine im Durchmesser verstellbare Ausrichtbohrung.

Insbesondere bildet die Ausrichteinrichtung einen Fixpunkt, wodurch das strukturierte Extrudat und der oder die stabförmigen Körper zwischen diesem Fixpunkt und dem in Produktionsrichtung vorne liegenden Ende des Führungskanals bzw. dem in Produktionsrichtung vorne liegenden Ende der Austrittsdüse exakt in axialer Richtung ausgerichtet und damit die Anordnung der stabförmigen Körper in einer vordefinierten Position im strukturierten Extrudat zusätzlich verbessert wird.

Die Ausrichteinrichtung kann durch Merkmale der Abzugseinrichtung oder einer Rolleneinrichtung realisiert sein. D.h. die Ausrichteinrichtung kann integraler Bestandteil einer der beiden Einrichtungen sein.

Die Abzugseinrichtung ist ausgebildet, um das strukturierte Extrudat aus der Vorrichtung abzuführen und/oder dieses durch die Vorrichtung hindurchzuführen. Mittels der Abzugseinrichtung kann sichergestellt werden, dass das strukturierte Extrudat und insbesondere zumindest ein darin angeordneter stabförmiger Körper permanent unter Spannung gehalten wird, sodass diese nicht durchhängen, wodurch ein gleichmäßiger Durchmesser über die komplette Länge des strukturierten Extrudats und eine gleichbleibende Anordnung des bzw. der stabförmigen Körper im strukturierten Extrudat gewährleistet wird.

Die Abzugseinrichtung ist vorzugsweise als Raupenabzug, der mit kettenartigen bzw. profilierten Elementen versehen ist, oder als Bandabzug mit Transportbändern ausgebildet.

Alternativ kann auch ein Trommelabzug, ein linearer Abzug mit Schlitten oder ein Walzenabzug vorgesehen sein. Der Abzug ist insbesondere derart ausgebildet, dass das strukturierte Extrudat durch den Abzugvorgang möglichst wenig verfestigt und/oder gedehnt wird, um die Form des strukturierten Extrudats durch den Abzug möglichst nicht zu verändern.

Die Einrichtung zum Zuführen stabförmiger Körper ist vorzugsweise ein Materialbaum, wobei auf dem Materialbaum vorzugsweise gebremste Spulen bzw. Rollen angeordnet sind, auf denen die stabförmigen Körper aufgewickelt sind. Durch das Abbremsen der Spulen werden beim Abrollen weniger Schwingungen in dem stabförmigen Körper erzeugt und diese gleichmäßig auf Spannung gehalten.

Vorzugsweise kann die Seitenwandung der Extrusionseinrichtung mit einer bzw. zwei bzw. drei radial umlaufend angeordneten Heizeinrichtungen versehen sein. Mittels der Heizeinrichtung ist es möglich, den Extrusionsraum auf einer konstanten Temperatur zu halten, um das Polymer in einem fließfähigen Zustand zu halten. Die Temperatur kann über eine mit Temperaturfühlern verbundene Steuereinrichtung entsprechen einstellbar sein.

Weiterhin kann am Extrusionsraum eine Drossel bzw. ein Ablassventil, vorzugsweise im Bereich der Seitenwandung, vorgesehen sein, um Polymer aus dem Extrusionsraum abzuführen. Durch dieses Bauteil wird zum einen verhindert, dass degradiertes Polymer verarbeitet wird und zum anderen besteht durch das Abführen des Polymers die Möglichkeit, dieses in Bewegung zu halten bzw. das Polymer zu beschleunigen, um eine Stagnation zu verhindern. Die Sicherstellung eines ausreichenden Polymerflusses ist angesichts der im strukturierten Extrudat enthaltenen nur sehr geringen Polymermengen sehr wichtig, um einen Abbau des Polymers im Extruder zu verhindern.

Ein zentraler Zuführkanal kann alternativ zum Zuführen von Luft ausgebildet sein. Weiterhin können mehrere dezentrale bzw. periphere Zuführkanäle, die gleich beabstandet radial umlaufend um den zentralen Zuführkanal angeordnet sind, zum Zuführen stabförmiger Körper vorgesehen sein. Der zentrale Zuführkanal ist in diesem Fall mit einer Einrichtung zum Zuführen von Druckluft verbunden, um bei der Extrusion einen Stützluftdruck zum Ausbilden eines Schlauch- oder Katheterlumens im strukturierten Extrudat, insbesondere in einem Schlauch oder Katheter aufzubauen.

Mittels einer derartigen Pinole mit Zuführkanälen mit den oben genannten Eigenschaften ist es möglich, Schläuche oder Katheter zu extrudieren, die in die Schlauch- oder Katheterwandung eingebettete stabförmige Körper aufweisen.

Die Anordnung der peripheren Zuführkanäle definiert die Position der stabförmigen Körper bezüglich des Lumens und deren relative Anordnung zueinander und somit auch deren Position in der Schlauch- oder Katheterwandung.

Die erfindungsgemäße Vorrichtung kann zum parallelen Herstellen mehrerer strukturierter Extrudate ausgebildet sein.

Im Folgenden werden die Vorteile der erfindungsgemäßen Vorrichtung gegenüber der aus dem Stand der Technik bekannten Vorrichtung erläutert.

Mittels der erfindungsgemäßen Vorrichtung wird eine gute Benetzung der stabförmigen Körper bei gleichzeitig bestmöglicher geometrischer Anordnung der stabförmigen Körper im Extrudat sichergestellt.

Die Zuführung der stabförmigen Körper in den Extrusionsraum wird erheblich vereinfacht.

Weiterhin werden lange Strecken, in denen die stabförmigen Körper an Kanalwandungen der Vorrichtung reiben, mit dem erfindungsgemäßen Aufbau vermieden.

Weiterhin lassen sich die Zuführkanäle der Pinole sehr leicht reinigen.

Durch den einfachen Aufbau ist es möglich, immer wieder die gleichen strukturierten Extrudate zu reproduzieren, wobei die lineare Ausrichtung der stabförmigen Körper durch die Führungskanäle quasi von selbst erfolgt.

Für jeden stabförmigen Körper ist ein Führungskanal vorgesehen, sodass sichergestellt ist, dass die stabförmigen Körper am in Produktionsrichtung vorne liegenden Ende im Bereich vor der Pinole in korrekter geometrischer Anordnung vorliegen.

Die erfindungsgemäße Vorrichtung ist für die sehr kleinen Dimensionen medizinischer Instrumente, insbesondere Katheter und Führungsdrähte geeignet. Würde man die einzelnen stabförmigen Körper einfach nur in die Pinole einführen und mit Polymer ummanteln, so würde keine brauchbare geometrische Anordnung der einzelnen stabförmigen Körper in den medizinischen Instrumenten erreicht. Dies liegt zum einen an der zufälligen Verteilung der stabförmigen Körper und an den daraus resultierenden zu großen oder zu kleinen Abständen der einzelnen stabförmigen Körper zueinander bzw. zwischen den peripheren stabförmigen Körpern und einem zentralen stabförmigen Körper. Zum anderen käme es zu keiner gleichmäßigen Überdeckung der peripheren stabförmigen Körper durch das Polymer. Gegebenenfalls könnten sogar die peripheren stabförmigen Körper radial aus dem strukturierten Extrudat herausragen bzw. freigelegt werden und das medizinische Instrument wäre unbrauchbar.

Im Folgenden werden stabförmige Körper gemäß einem weiteren Aspekt der vorliegenden Erfindung beschrieben.

Um die mechanischen Festigkeitseigenschaften, wie z.B. die Zug- und Druck- sowie die Biegesteifigkeit eines vorstehend genannten strukturierten Extrudats zu verbessern, sind gemäß diesem weiteren Aspekt der vorliegenden Erfindung ein oder mehrere stabförmige Körper vorgesehen, aus denen mittels der erfindungsgemäßen Vorrichtung zum Extrudieren eines strukturierten Extrudats ein strukturiertes Extrudat mit verbesserten mechanischen Eigenschaften herstellbar ist.

Solche erfindungsgemäßen stabförmigen Körper weisen gegenüber dem bekannten runden Querschnitt eine andere Querschnittsgeometrie auf. Ein solcher konturierter Querschnitt des stabförmigen Körpers ist dann z.B. elliptisch oder oval bzw. bohnenförmig oder trapezförmig oder drei- oder vier- oder vieleckig ausgebildet.

Die Kanten der vorstehend genannten konturierten Querschnitte können im Querschnitt gerade oder gekrümmt bzw. gegenüber einer Ebene quer zu einer Längsrichtung des stabförmigen Körpers konkav oder konvex ausgebildet sein.

Ein solcher stabförmiger Körper und insbesondere auch ein aus einem oder mehreren solchen stabförmigen Körpern mittels der erfindungsgemäßen Vorrichtung hergestelltes medizinisches Instrument weist eine gegenüber einem stabförmigen Körper mit rundem Querschnitt und ähnlichem Durchmesser bzw. einem entsprechenden medizinischen Instrument umfassend runde stabförmige Körper wesentlich erhöhte Zug-, Druck- und Biegesteifigkeit auf.

Einem solchen Aufbau liegt die Erkenntnis zu Grunde, dass durch eine Erhöhung des Anteils stabförmiger Körper im Querschnitt des medizinischen Instruments die mechanische Festigkeit erheblich verbesserbar ist. Dabei kann die zur Verfügung stehende Querschnittsfläche im medizinischen Instrument durch Veränderung der Querschnittsgeometrie der stabförmigen Körper mit nicht-metallischen Filamenten gegenüber einem kreisförmigen Querschnitt der stabförmigen Körper erhöht werden. Dies gilt insbesondere für die peripheren stabförmigen Körper. Bei runden stabförmigen Körpern ist ein signifikanter Anteil der Querschnittsfläche eines medizinischen Instruments nicht mit nicht-metallischen Fasern, sondern mit Hüllpolymer ausgefüllt. Um eine vermehrte Füllung der Querschnittsfläche mit nicht-metallischen Fasern zu erreichen, können die stabförmigen Körper mit einer optimierten Querschnittsgeometrie verwendet werden.

Dabei kann sich die Form des zentralen stabförmigen Körpers von der Form der peripheren stabförmigen Körper unterscheiden.

Die Verwendung elliptischer peripherer stabförmiger Körper ermöglicht eine höhere Steifigkeit in Längsrichtung des medizinischen Instruments als bei Verwendung runder stabförmiger Körper. Eine weitere Erhöhung der Steifigkeit des medizinischen Instruments ist durch die Verwendung trapezförmiger stabförmiger Körper zu erreichen. Dabei ist zu beachten, dass eine ausreichende Abstandsfläche zwischen den einzelnen stabförmigen Körpern verbleiben muss, so dass diese durch das Hüllpolymer geometrisch im Querschnitt fixiert und miteinander verbunden werden. Eine Erhöhung der mechanischen Steifigkeit in Längsrichtung des medizinischen Instruments kann auch durch eine Optimierung der geometrischen Form des zentralen stabförmigen Körpers erreicht werden.

Durch das vorstehend beschriebene System der modularen Modifizierung der geometrischen Form der stabförmigen Körper in einem medizinischen Instrument kann die mechanische Steifigkeit in dessen Längsrichtung inkrementell und in feinen Stufen gegenüber der Ausführung nur mit runden stabförmigen Körpern erhöht und den medizinischen und technischen Anwendungsanforderungen angepasst werden. Auf diese Weise kann eine kommerziellen metallbasierten Führungsdrähten angenäherte mechanische Steifigkeit in Längsrichtung für steife und super-steife Führungsdrähte erreicht werden, ohne Metallseelen zu verwenden, welche in MR-kompatiblen medizinischen Instrumenten nicht eingesetzt werden können.

Im Folgenden wird der Aufbau eines solchen stabförmigen Körpers beschrieben, der sich dadurch auszeichnet, dass der stabförmigen Körper im Querschnitt elliptisch oder oval bzw. bohnenförmig oder trapezförmig oder drei- bzw. vier- bzw. vieleckig ausgebildet ist:
Merkmale eines solchen stabförmigen Körpers 29 sind in der WO 2007/000148, der WO 2015/161927 und der WO 2012/052159 offenbart, auf die hiermit voll inhaltlich Bezug genommen wird. Die nachfolgenden technischen Merkmale der stabförmigen Körper sind in beliebiger Weise kombinier- und/oder austauschbar.

Ein stabförmiger Körper umfasst eine oder mehrere nicht-metallische Filamente und einen nicht-ferromagnetischen Matrixwerkstoff bzw. ein Matrixmaterial, wobei der Matrixwerkstoff das oder die Filamente umschließt und/oder miteinander verklebt, und wobei in den Matrixwerkstoff eine Dotierung aus magnetresonanztomografisch Artefakte erzeugenden Partikeln eingebracht ist. Die Kombination aus dem Matrixwerkstoff mit dem oder den nicht-metallischen Filament(en) erzielt auf überraschend einfache Weise metallähnliche Steifigkeits-, Biegsamkeits- und Elastizitätseigenschaften.

Die den stabförmigen Körper bildenden Filamente sind leicht und kostengünstig herstellbar, dabei ausreichend stabil und können Druck- und Zugkräfte sowie Drehmomente übertragen. Durch die Dotierung des zum Umschließen eines einzelnen Filamentes oder zum Verkleben mehrerer Filamente ohnehin erforderlichen Matrixwerkstoffes ist eine einfache und effektive Möglichkeit geschaffen, den stabförmigen Körper in der MRT darzustellen und gleichzeitig eine dem heute üblichen Standard entsprechende Handhabbarkeit zu gewährleisten.

Aus diesem Grundelement werden, wie weiter unten beschrieben ist, die eigentlichen Instrumente wie Katheter, Führungsdrähte etc. aufgebaut.

Die Filamente können aus Kunststoff und/oder Glasfaser ausgebildet sein. Derartige Filamente können leicht und preiswert, in großen Längen, mit unterschiedlichsten Querschnitten und Dicken hergestellt werden. Insbesondere Glasfaser verfügt über geringste Dehnungen, weshalb eine sehr direkte Kraft- und Momentenübertragung möglich ist.

Der Matrixwerkstoff kann aus Epoxidharz ausgebildet sein. Epoxidharze stehen in einer großen Fülle von Eigenschaften zur Verfügung und die zur Verarbeitung erforderlichen Geräte sind ausgereift.

Die Stäbe können entlang ihrer Längsachse kontinuierlich mit magnetresonanztomografisch Artefakte erzeugenden Partikeln dotiert sein. Hierdurch wird der Stab auf seiner gesamten Länge in der MRT gut sichtbar.

Für manche Anwendungen kann es jedoch auch bevorzugt sein, den Stab entlang seiner Längsachse diskontinuierlich, insbesondere abschnittsweise mit magnetresonanztomografisch Artefakte erzeugenden Partikeln zu dotieren. Dies trifft insbesondere für die Spitzen der Stäbe zu, die in manchen Fällen besonders gut sichtbar sein sollen.

Innerhalb der Stäbe können die Filamente parallel verlaufend angeordnet sein. Hierdurch wird eine besonders einfache Verarbeitung unterstützt.

Die Filamente können nach besonderes bevorzugten Ausführungsformen jedoch auch miteinander verflochten, verwoben, vernetzt, verdrillt oder wendelförmig verlaufend angeordnet sein, um bestimmte gewünschte Eigenschaften, insbesondere mechanische Eigenschaften zu erzielen.

Vorteilhaft ist es, wenn die Masse eines einzelnen Partikels im Mikro- bis Nanogramm-Bereich liegt und aufgrund der im Vergleich zum Matrixwerkstoff geringen Menge die äußere Form, Stabilität und die Torquierungseigenschaften des Stabs im Wesentlichen nicht beeinflusst.

Typische bevorzugte Größen der Stäbe liegen im Bereich eines Durchmessers zwischen 0,005 mm und 5 mm, vorzugsweise zwischen 0,1 und 1 mm.

Aus den beschriebenen Stäben kann ein zylindrischer Verbindungskörper ("Zylinder"), insbesondere ein Führungsdraht, ausgebildet werden, und zwar dergestalt, dass mindestens ein Stab von einem nicht-ferromagnetischen Matrixwerkstoff umschlossen wird bzw. mehrere Stäbe mit einem nicht-ferromagnetischen Matrixwerkstoff verklebt und/oder umschlossen werden.

Auf diese Weise können mit ein und demselben Element (dem Stab) und dem Matrixwerkstoff sehr leicht und kostengünstig Zylinder unterschiedlichster Geometrien und mechanischer sowie MRT-Eigenschaften aufgebaut werden.

Der Zylinder kann z. B. besonderes einfach aus Stäben gleichen Durchmessers bestehen oder - nach einer anderen Ausführungsform - aus Stäben unterschiedlichen Durchmessers. Im letzteren Falle können insbesondere um einen ersten Stab herum zweite Stäbe geringeren Durchmessers angeordnet sein.

Die einzelnen Stäbe können - wie zuvor die Filamente - miteinander verflochten, verwoben, vernetzt, verdrillt oder wendelförmig verlaufend angeordnet sein.

Besonders bevorzugt ist es, wenn die Zylinder Stäbe unterschiedlicher magnetresonanztomografischer Eigenschaften aufweisen. So kann ein und der selbe Zylinder (z. B. als Führungsdraht) in unterschiedlichen MRT-Sequenzen (zum Beispiel zur spezifischen Darstellung von Fettgewebe, Muskelgewebe usw.) gleich gut beobachtet werden.

Vorteilhaft ist es, die äußere Oberfläche des Zylinders hydrophil zu beschichten und ihn damit körperverträglich zu machen.

Aus den beschriebenen Stäben können jedoch auch andere Instrumente gefertigt werden, insbesondere ein schlauchförmiger Verbindungskörper ("Katheter"). Dieser besteht aus mindestens einem Stab, der von einem nicht-ferromagnetischen Matrixwerkstoff umschlossen wird und/oder mehrerer Stäbe miteinander verklebt und/oder umschließt.

Nach einer Ausführungsform des Katheters besteht dieser aus mehreren, in seiner Wandung auf dem Umfang radiär verteilt eingebetteten Stäben. Diese können insbesondere zur Erzielung symmetrischer Eigenschaften regelmäßig radiär verteilt eingebettet sein.

Aus dem gleichen Grunde können die den Katheter bildenden Stäbe gleichen Durchmesser aufweisen, in besonderen Fällen können aber auch Stäbe unterschiedlichen Durchmessers zur Anwendung kommen.

Ebenso wie bei den Zylindern / Führungsdrähten können auch bei dem Katheter die Stäbe miteinander verflochten, verwoben, vernetzt, verdrillt oder wendelförmig verlaufend angeordnet sein, die Stäbe unterschiedliche magnetresonanztomografische Eigenschaften aufweisen und seine äußere Oberfläche hydrophil beschichtet sein.

Gemäß einem weiteren Aspekt kann der stabförmige Körper einen Zentralabschnitt und einen Peripherabschnitt umfassen, wobei der Zentralabschnitt im Zentrum des stabförmigen Körpers angeordnet ist und vom Peripherabschnitt umschlossen ist. Sowohl der Zentralabschnitt als auch der Peripherabschnitt erstrecken sich über im Wesentlichen die gesamte Länge des stabförmigen Körpers. Der Zentralabschnitt weist zumindest ein in ein nicht-ferromagnetisches Matrixmaterial eingebettetes, nicht-metallisches Faserbündel auf. Das Matrixmaterial ist mit Markerpartikeln dotiert. Der Peripherabschnitt weist ein undotiertes, nicht-ferromagnetisches Matrixmaterial auf. Der Durchmesser des Zentralabschnitts ist kleiner oder gleich 0,2 mm, bevorzugt kleiner oder gleich 0,15 mm und noch mehr bevorzugt kleiner oder gleich 0,1 mm und insbesondere kleiner oder gleich 0,08 mm.

Dadurch, dass lediglich das Matrixmaterial des sehr kleinen Zentralabschnitts mit MR-Markerpartikeln dotiert ist, wird bei der Magnetresonanztomographie ein besonders schmales und scharfes Artefakt erzeugt.

Eine derart konzentrierte Anordnung einer gegenüber dem Stand der Technik geringeren Menge von MR-Markerpartikeln ist vorteilhaft gegenüber derjenigen mit einer höheren Menge von MR-Markerpartikeln, die im gesamten stabförmigen Körper verteilt sind, da die Voxel entlang der konzentrierten Anordnung von MR-Partikeln geschwärzt werden, aber diese Voxel den gleichen Schwärzungsgrad aufweisen wie bei einer größeren Menge an MR-Markerpartikeln, die über einen entsprechend größeren Bereich verteilt ist. Dadurch werden in der Breite weniger Voxel geschwärzt und ein schmaleres Abbild des medizinischen Instruments erreicht, so dass weniger Zielgewebe durch Schwärzung im MRT-Bild überdeckt werden kann.

Daher ist es mit einem stabförmigen Körper mit MR-Marker-Dotierung nur im Zentralabschnitt möglich, in der Magnetresonanztomographie ein annähernd ebenso schmales Abbild zu erzeugen wie mit einem metallischen Führungsdraht in einem Röntgenbildgebungsverfahren. Dies gilt insbesondere, wenn das medizinische Instrument lediglich einen einzigen dotierten stabförmigen Körper aufweist oder bei mehreren stabförmigen Körpern nur der zentral angeordnete stabförmige Körper MR-Marker aufweist. Vorzugsweise ist dann der mit zentrierter Anordnung von Markerpartikeln ausgebildete stabförmige Körper im Zentrum des Führungsdrahtes angeordnet. Hierdurch wird der Abstand zwischen dem dotierten Zentralabschnitt und der Oberfläche des Führungsdrahtes maximal gehalten. Dies führt dazu, dass Wasser- oder Fettmoleküle im zu untersuchenden Körper nicht näher als an die Oberfläche des Führungsdrahtes an den Zentralabschnitt heran gelangen können. Dadurch wird die Resonanz zwischen den MR-Markern und den Wassermolekülen gering gehalten, wodurch die von den MR-Markern erzeugten Artefakte klein sind und sich der Führungsdraht als schmaler Streifen in einem MR-Bildgebungsverfahren darstellt.

Zudem hat sich gezeigt, dass aufgrund der Konzentration der MR-Marker auf den Zentralabschnitt der Anteil der MR-Marker im Matrixmaterial über einen großen Bereich variieren kann, ohne dass dies nennenswert die Darstellung des Führungsdrahtes im MR-Bildgebungsverfahren beeinflusst. Bei Verwendung von Eisenpartikeln mit einer Korngröße von 0 bis 20 µm wurde bei einem Gewichtsverhältnis von ca. 1:5 bis ca. 1:30 zwischen Markern und Matrixmaterial im Wesentlichen die gleiche Darstellung erzielt. Es hat sich gezeigt, dass die lokale Konzentration auf einen an sich möglichst kleinen Bereich, d.h. den Zentralabschnitt wesentlich mehr Einfluss auf die Darstellung im MR-Bildgebungsverfahren hat als die Anteile an Markern im Matrixmaterial.

Der stabförmige Körper weist vorzugsweise einen Durchmesser von nicht mehr als 0,75 mm und insbesondere einen Durchmesser von 0,5 mm auf. Mit der Erfindung werden erstmals derartig dünne stabförmige Körper mit einer Struktur geschaffen, die einen Zentralabschnitt und einen Peripherabschnitt aufweisen.

Ein stabförmiger Körper ist ein einteiliger Vollmaterialkörper. Es ist kein rohr- oder schlauchförmiger Hohlkörper.

Vorzugsweise besteht der stabförmige Körper aus einem homogenen Matrixmaterial. Das Matrixmaterial des Peripherabschnitts und des Zentralabschnitts bestehen somit aus dem gleichen Materialtyp. Der bevorzugte Materialtyp ist Epoxidharz. Andere Materialtypen können aus anderen vorzugsweise auch chemisch reaktiven und funktionellen Polymeren ausgebildet sein. Geeignete Beispiele sind radikalische oder ionische Quervernetzungen von Polymeren, wie z.B. ungesättigten Polyestern, Veretherungen und Veresterungen von Polysachariden, Hydrolysen von Polyvinylestern oder Acetalisierungen von Polyvinylalkohol.

Vorzugsweise sind die nicht-metallischen Fasern im Peripherabschnitt etwa gleichmäßig bzgl. des Querschnitts angeordnet. Hierdurch wird auch bei einem dünnen oder kleinvolumigen Peripherabschnitt eine hohe Torsions- und Biegefestigkeit erzielt.

Es hat sich auch gezeigt, dass durch die Konzentration auf den Zentralabschnitt die gesamte Menge an MR-Marker sehr gering ist und trotzdem eine sehr gute Abbildung erzielt wird.

Auf diese Weise wird eine bestmögliche Darstellung eines medizinischen Instruments, insbesondere eines Führungsdrahtes, das einen derartigen stabförmigen Körper mit Dotierung nur im Zentralabschnitt umfasst, erzielt.

Zusätzlich zu den im Zentralabschnitt der stabförmigen Körper enthaltenen MR-Markerpartikeln kann auch ein separater MR-Spitzenmarker am distalen Endbereich der medizinischen Instrumente aufgebracht werden, der ein breiteres Artefakt, z.B. mit der zwei- bis dreifachen Breite des Artefakts über die gesamte Länge des medizinischen Instruments, im MRT-Bild erzeugt. Mit solch einem Spitzenmarker kann die Spitze des medizinischen Instruments und damit dessen distales Ende eindeutig im MRT-Bild identifiziert werden. Ist der Spitzenmarker nicht sichtbar im MRT-Bild, so befindet sich die Spitze nicht in der visualisierten Schicht der MRT-Aufnahme und die Schichteinstellung muss nachgestellt werden.

Der MR-Spitzenmarker wird bspw. durch Auftragen einer selbsthärtenden Polymerlösung, die MR-Markerpartikel enthält, hergestellt. Nach dem Aushärten bildet die Polymerlösung eine Schicht, die sich vorzugsweise über einen Längsbereich von einigen µm bis zu einigen mm erstreckt. Die Schicht kann alleine an einer Stirnfläche des stabförmigen Körpers aufgebracht sein, sodass die Längserstreckung der Schichtdicke entspricht. Die Schicht kann jedoch auch auf die Mantelfläche des stabförmigen Körpers aufgetragen sein, wobei dann die Längserstreckung vorzugsweise nicht länger als 5 mm und insbesondere nicht länger als 3 mm ist. Die Polymerlösung kann z.B. aus PEBAX oder einem Klebstoff bestehen. Als MR-Markerpartikel können alle hierin offenbarten MR-Marker verwendet werden, wobei Eisen-Markerpartikel bevorzugt werden. Der Spitzenmarker kann mit weiteren Schichten wie z.B. einer Hüllmaterialschicht überzogen werden. Bevorzugt ist eine solche weitere Überdeckung mit Polymermaterial.

Am distalen Ende der medizinischen Instrumente können auch mehrere Spitzenmarker aufgebracht werden, vorzugsweise in einem definierten Abstand voneinander. Auf diese Weise kann eine Messfunktion in das medizinische Instrument eingebaut werden, um die Länge z.B. einer Gefäßstenose im Körper ausmessen zu können.

Die Größe und insbesondere die Breite des Spitzenmarker-Artefakts ist abhängig von der absoluten aufgetragenen Menge der MR-Markerpartikel und von der Schichtdicke der über dem Spitzenmarker befindlichen Material-/Polymerschicht, weil diese den Abstand zu den umgebenden Wasser- oder Fettmolekülen bestimmt.

Ein Faserbündel kann mindestens ein langgestrecktes oder mehrere langgestreckte Fasern und vorzugsweise mehrere langgestreckte Fasern umfassen, die parallel angeordnet oder miteinander verflochten oder verdrillt sind. Dadurch, dass das Faserbündel mindestens eine langgestreckte oder mehrere langgestreckte Fasern umfasst, verleiht das Faserbündel dem stabförmigen Körper eine hohe Festigkeit in longitudinaler Richtung. Durch eine derartige geordnete Ausbildung und Anordnung der Faserbündel im stabförmigen Körper wird eine bessere Produktqualität erreicht.

Das Faserbündel im Zentralabschnitt kann ein ht-Faserbündel sein und das Faserbündel im Peripherabschnitt kann ein Glasfaserbündel sein.

Ein ht-Faserbündel ist ein hochfestes Faserbündel. Typische Beispiele für ht-Faserbündel sind Aramidfasern und UHMWPE-Fasern (Ultra High Molecular Weight Polyethylen Fibers). ht-Faserbündel haben eine Zug- bzw. Reißfestigkeit von zumindest 20 cN/tex. Optional haben die ht-Faserbündel eine Zug- bzw. Reißfestigkeit von zumindest 32 cN/tex und insbesondere von zumindest 30 cN/tex.

Ein ht-Faserbündel ist hochflexibel bzw. biegsam und weist eine hohe Zug- bzw. Reißfestigkeit auf. Auf diese Weise ist sichergestellt, dass selbst wenn die stabförmigen Körper im menschlichen oder tierischen Körper während der medizinischen Intervention brechen, die gebrochenen Teile immer noch durch das ht-Faserbündel miteinander verbunden bleiben, wodurch das medizinische Instrument sicher entfernbar ist. Zudem verleiht das in das Matrixmaterial eingebettete ht-Faserbündel dem stabförmigen Körper eine gewisse Steifigkeit.

Glasfaserbündel sind steifer als ht-Faserbündel, so dass ein medizinisches Instrument, das sowohl ht-Faserbündel als auch Glasfaserbündel aufweist, bevorzugt ist.

Ein stabförmiger Körper, der sowohl ht-Faserbündel als auch Glasfaserbündel aufweist, kann hinsichtlich seiner Steifigkeit und Flexibilität und insbesondere bezüglich seiner Torsionssteifigkeit optimal eingestellt werden.

Die Anordnung zumindest eines Glasfaserbündels im Peripherabschnitt ermöglicht die höchstmögliche Steifigkeit. Das zumindest eine periphere Glasfaserbündel gibt dem stabförmigen Körper die notwendige Kompressions- und Biegesteifigkeit. Dadurch, dass zumindest ein ht-Faserbündel zentral auf einer neutralen Linie angeordnet ist, mindert das zumindest eine ht-Faserbündel die Kompressions- und die Biegesteifigkeit des stabförmigen Körpers nur minimal. Als neutrale Linie, auch Nulllinie genannt, bezeichnet man in der technischen Mechanik im Rahmen der Elastostatik die Schicht eines Querschnitts eines stabförmigen Körpers, deren Länge sich bei einem Biegevorgang nicht ändert. Dort verursacht die Biegung keine Zug- oder Druckspannungen. Die Fläche verläuft durch den geometrischen Schwerpunkt der Querschnittsfläche des stabförmigen Körpers.

Das zumindest eine Faserbündel im Zentralabschnitt kann auch ein Glasfaserbündel sein und das zumindest eine Faserbündel im Peripherabschnitt ein ht-Faserbündel. Diese Anordnung ist dann optimal, wenn der stabförmige Körper einen geringen Anteil an Glasfaser und einen hohen Anteil an ht-Fasern haben soll. In dieser Ausführungsform wird ein überwiegend aus der flexibleren ht-Faser bestehender stabförmiger Körper in der Kompressions- und Biegesteifigkeit durch die Glasfasern verstärkt.

Es ist vorteilhaft, die im größeren Anteil enthaltenen Fasern an der Oberfläche des stabförmigen Körpers und die im kleineren Anteil enthaltenen Fasern im Inneren anzuordnen, um eine homogene Oberfläche zu erhalten. Die Produktqualität wird dadurch wesentlich erhöht.

Die nicht-metallischen Faserbündel sind elektrisch nicht-leitende Fasern bzw. Filamente, so dass diese während der Magnetresonanztomographie verwendbar sind. Demgemäß schließt der Begriff "nicht-metallisches Faserbündel", wie er in der vorliegenden Beschreibung verwendet wird, jegliche elektrisch leitfähige Fasern, wie zum Beispiel dünne Metalldrähte oder ein Karbonfilament aus.

Vorzugsweise ist ein Faserbündel aus mehreren Fasern ausgebildet. Ein derartiges Faserbündel wird im Englischen als "roving" bezeichnet.

Wenn die Fasern des Faserbündels miteinander verdrillt sind, bilden diese ein Garn aus. Ein derartiges Faserbündel wird im Englischen als "yarn" bezeichnet.

Alle Faserbündel im stabförmigen Körper können ht-Faserbündel sein. Ein derart ausgebildeter stabförmiger Körper weist bestmögliche Eigenschaften hinsichtlich der Reißfestigkeit auf.

Weiterhin können alle Faserbündel im stabförmigen Körper Glasfaserbündel sein. Ein derartiger stabförmiger Körper weist bestmögliche Eigenschaften hinsichtlich der Kompressions- und Biegesteifigkeit auf.

Das nicht-ferromagnetische Matrixmaterial im Zentralabschnitt und im Peripherabschnitt kann das gleiche nicht-ferromagnetische Matrixmaterial sein. Als Matrixmaterial ist vorzugsweise Epoxidharz vorgesehen.

Die Markerpartikel im Zentralabschnitt sind vorzugsweise MR-Markerpartikel.

Im Zentralabschnitt können ein oder mehrere Faserbündel vorgesehen sein und im Peripherabschnitt können radial umlaufend um das Faserbündel des Zentralabschnitts und in etwa gleich beabstandet voneinander angeordnet zumindest drei bzw. vier bzw. fünf bzw. sechs bzw. sieben bzw. acht bzw. neun bzw. zehn bzw. elf bzw. zwölf Faserbündel vorgesehen sein.

Ein medizinisches Instrument, bspw. ein Führungsdraht oder ein Katheter oder eine Seele für eine Lead-Sonde, insbesondere ein Mikro-Führungsdraht, kann einen stabförmigen Körper gemäß der vorstehenden Beschreibung umfassen.

Ein derartiger Mikro-Führungsdraht oder eine derartige Seele aus nur einem stabförmigen Körper erzielt eine maximale Biege- und Kompressionssteifigkeit, wobei durch das ht-Faserbündel die Reißfestigkeit sichergestellt wird.

Dadurch, dass lediglich das Matrixmaterial des Zentralabschnitts des stabförmigen Körpers des Führungsdrahtes mit MR-Markerpartikeln dotiert ist, wird bei der Magnetresonanztomographie ein besonders schmales und scharfes Artefakt erzeugt. Bzgl. der Vorteile des schmalen Artefakts wird auf die Erläuterungen zum erfindungsgemäßen stabförmigen Körper verwiesen.

Im Bereich eines als Spitze ausgebildeten distalen Endes eines Führungsdrahtes oder einer Seele kann der Anteil von Glasfasern des Faserbündels kleiner sein als im übrigen Führungsdraht.

Dadurch weist ein derartiger Mikro-Führungsdraht oder eine derartige Seele eine flexible Spitze am distalen Ende auf, die dadurch ausgebildet wird, dass dort der Anteil an Glasfasern geringer als im restlichen Teil des Führungsdrahts oder der Seele ist bzw. sehr gering ist bzw. dass dort keine Glasfasern vorhanden sind. Somit enthält die flexible Spitze vorzugsweise nur noch ht-Fasern bzw. ein ht-Faser-Matrix-Komposit, so dass sie plastisch verformbar ist. Dies ist in der klinischen Praxis für die schnelle Anpassung der flexiblen Spitze an die Gefäßstrukturen in einer Zielregion erwünscht. Die flexible Spitze kann vom Arzt selbst, ohne Zuhilfenahme von Wasserdampf und/oder anderweitiger Erwärmung, in die gewünschte Form gebracht werden. Somit ist ein derartiger Mikro-Führungsdraht äußerst flexibel einsetzbar und kostengünstig herstellbar, da keine verschiedenen Spitzenformen vorgefertigt werden müssen. Zudem erlaubt die freie plastische Verformbarkeit eine wesentlich bessere Anpassung an die Bedürfnisse des anwendenden Arztes.

Wenn der Anteil an Glasfasern dagegen wesentlich höher ist als der an ht-Fasern, können derartige flexible Spitzen durch Wärmeeinwirkung in eine definierte vorgeformte und nicht einfach zu verändernde Krümmung bzw. mit einem gewünschten Krümmungsradius beaufschlagt werden.

Ein stabförmiger Körper für einen Mikro-Führungsdraht umfasst vorzugsweise im Zentralabschnitt ein oder zwei ht-Faserbündel und mehrere Glasfaserbündel im Peripherabschnitt.

Weiterhin kann der Mikro-Führungsdraht von einem Hüllmaterial umgeben sein, z.B. Polyamid oder Polyurethan. Das Hüllmaterial kann auch z.B. durch einen Schrumpfschlauch (vorzugsweise aus PTFE [Polytetrafluorethylen] oder FEP [Fluoroethylen-Propylen]) ausgebildet werden. Ein Schrumpfschlauch verbessert die Eigenschaften hinsichtlich der Reißfestigkeit des Führungsdrahts gegenüber einem z.B. durch Extrusion aufgebrachten Hüllmaterial.

Durch die Ausbildung der Oberfläche mit einem eine möglichst geringe Reibung aufweisenden Material, z.B. PTFE, werden zudem die mechanischen Eigenschaften des betreffenden medizinischen Instruments weiter verbessert. Z.B. weisen Führungsdrähte aus faserverstärkten Polymeren (FVP) gegenüber Führungsdrähten mit Metallseelen aufgrund der anderen Materialeigenschaften zumeist nicht ganz optimale Torquierungseigenschaften auf. Durch das Vorsehen der minimalen Friktion an z.B. einer Blutgefäßwand oder einer Katheterwand resultierend aus der reibungsarmen Führungsdrahtoberfläche sinken die mechanischen Anforderungen an die Drehsteifigkeit eines solchen FVP-basierten Führungsdrahts.

Hinsichtlich der Mikro-Führungsdrähte ist festzustellen, dass sich fast kein Abstand der MR-Markerpartikel von umgebenden Wasser- oder Fettmolekülen realisieren lässt, wenn der gesamte stabförmige Körper dotiert ist. Das bedeutet, die MR-Markerpartikel sind bei aus dem Stand der Technik bekannten Mikro-Führungsdrähten annähernd direkt benachbart zu umgebenden Wasser- oder Fettmolekülen angeordnet. Dies führt dazu, dass das Artefakt breiter als erwünscht wird, weil nicht, wie bei Standard- und Stiff-Führungsdrähten, die aus mehreren stabförmigen Körpern zusammengesetzt sind (siehe WO 2012/052159 A2), nur der zentrale stabförmige Körper dotiert werden kann, so dass dieser einen größeren Abstand von umgebenden Wasser- oder Fettmolekülen erhält.

Ein Mikro-Führungsdraht erzeugt somit ein sehr schmales und sehr scharfes Artefakt, da die MR-Markerpartikel im stabförmigen Körper durch die konzentrierte zentrale Anordnung ausreichend weit weg von umgebenden Wasser- oder Fettmolekülen angeordnet sind und somit nur die minimal mögliche Anzahl Voxel geschwärzt wird.

Gemäß einem weiteren Aspekt umfasst ein Führungsdraht zumindest einen zentralen stabförmigen Körper mit Dotierung nur im Zentralabschnitt mit MR-Marker gemäß der vorstehenden Beschreibung und zumindest einen peripheren stabförmigen Körper, wobei der zentrale stabförmige Körper im Zentrum des Führungsdrahtes angeordnet ist und wobei sich der zentrale stabförmige Körper und der periphere stabförmige Körper im Wesentlichen über die gesamte Länge des Führungsdrahtes erstrecken. Der periphere stabförmige Körper weist zumindest ein in ein undotiertes, nicht-ferromagnetisches Matrixmaterial eingebettetes, nicht-metallisches Faserbündel auf. Der zentrale stabförmige Körper und der periphere stabförmige Körper sind in ein nicht-ferromagnetisches Hüllmaterial eingebettet. Zusätzlich kann zur Erhöhung der Reißfestigkeit und für die Erzielung einer optimal glatten Oberfläche ein Schrumpfschlauch aufgebracht werden.

Ein derart erfindungsgemäß ausgebildeter Führungsdraht erzeugt somit ein sehr schmales und sehr scharfes Artefakt, da die MR-Markerpartikel im zentralen stabförmigen Körper durch die konzentrierte zentrale Anordnung optimal konzentriert und optimal weit weg vom umgebenden Wasser angeordnet sind.

Der zentrale stabförmige Körper kann einen Zentralabschnitt und einen Peripherabschnitt aufweisen, wobei der Zentralabschnitt im Zentrum des stabförmigen Körpers angeordnet ist und vom Peripherabschnitt umschlossen ist, wobei sich sowohl der Zentralabschnitt als auch der Peripherabschnitt über die gesamte Länge des stabförmigen Körpers erstrecken, und der Zentralabschnitt zumindest ein Glasfaser-Faserbündel aufweist und der Peripherabschnitt zumindest ein ht-Faserbündel aufweist, wobei beide Faserbündel in ein nicht-ferromagnetisches Matrixmaterial eingebettet sind.

Das Faserbündel des peripheren stabförmigen Körpers kann ein Glasfaserbündel sein.

Der periphere stabförmige Körper kann einen Zentralabschnitt und einen Peripherabschnitt aufweisen, wobei der Zentralabschnitt im Zentrum des stabförmigen Körpers angeordnet ist und vom Peripherabschnitt umschlossen ist, wobei sich sowohl der Zentralabschnitt als auch der Peripherabschnitt über die gesamte Länge des stabförmigen Körpers erstrecken, und der Zentralabschnitt zumindest ein ht-Faserbündel aufweist und der Peripherabschnitt zumindest ein Glasfaserbündel aufweist, wobei beide Faserbündel in ein nicht-ferromagnetisches Matrixmaterial eingebettet sind.

Der Führungsdraht kann einen zentralen stabförmigen Körper und zumindest drei bzw. vier bzw. fünf bzw. sechs bzw. sieben bzw. acht bzw. neun bzw. zehn bzw. zwölf stabförmige Körper aufweisen.

Gemäß einem weiteren Aspekt ist ein Verfahren zur Herstellung eines stabförmigen Körpers gemäß der vorstehenden Beschreibung vorgesehen, wobei ein Zentralabschnitt zumindest eines nicht-metallischen Faserbündels mit einem mit MR-Markerpartikel dotierten, nicht-ferromagnetischen Matrixmaterial ausgebildet wird und auf dem Zentralabschnitt durch Imprägnieren zumindest eines nicht-metallischen Faserbündels mit undotiertem, nichtferromagnetischem Matrixmaterial ein Peripherabschnitt ausgebildet wird, so dass dieser den Zentralabschnitt umschließt.

Da in der erfindungsgemäßen Ausführung der stabförmigen Körper die MR-Markerpartikel im Inneren des stabförmigen Körpers angeordnet werden, kommen diese nicht an die Oberfläche des stabförmigen Körpers und beeinträchtigen den Pultrusionsprozess nicht, so wie es der Fall sein kann, wenn die MR-Markerpartikel an der Oberfläche des stabförmigen Körpers liegen und dadurch zu einem ungleichmäßigen Passieren des Ausformwerkzeugs führen können, welches Inhomogenitäten im Pultrudat hervorrufen kann. Je gleichmäßiger der Pultrusionsprozess abläuft, umso höher ist die Produktqualität.

Die geordnete Anordnung der Fasern, insbesondere wenn in einem stabförmigen Körper zwei oder mehr verschiedene Fasertypen enthalten sind, führt zu einer besseren und homogeneren Imprägnierung der Fasern mit dem Matrixmaterial und damit ebenfalls zu einer höheren Produktqualität.

Die Anordnung sämtlicher in einem medizinischen Instrument enthaltenen ht-Fasern in einem einzigen stabförmigen Körper verleiht diesem die bestmögliche Reißfestigkeit und damit eine verbesserte Produktqualität.

Entsprechend einem weiteren Aspekt umfasst ein stabförmiger Körper
- ein oder mehrere nicht-metallische Filamente und
- ein nicht-ferromagnetisches Matrixmaterial,
dadurch gekennzeichnet, dass das Matrixmaterial die Filamente umschließt und/oder miteinander verklebt,
und Markerpartikel, die in der Röntgen- oder Magnetresonanzbildgebung ein Signal erzeugen, wobei mindestens eines der nicht-metallischen Filamente eine ht-Faser ist.

Eine ht-Faser ist eine Faser mit hoher Zugfestigkeit. Typische Beispiele von ht-Fasern sind Aramid-Fasern und UHMWPE-Fasern (Ultra High Molecular Weight Polyethylen-Fasern). ht-Fasern haben eine Zugfestigkeit von mind 20 cN/tex. Optional haben die ht-Fasern eine Zugfestigkeit von mind. 23 cN/tex und insbesondere von mind. 30 cN/tex.

Eine ht-Faser ist hochflexibel und besitzt eine hohe Zugfestigkeit. Dadurch wird sichergestellt, dass selbst wenn ein stabförmiger Körper während der medizinischen Intervention im menschlichen oder tierischen Körper bricht, die gebrochenen Teile weiterhin durch die ht-Faser miteinander verbunden bleiben und sicher herausgezogen werden können.

Weiterhin verleiht die ht-Faser dem stabförmigen Körper eine gewisse Steifigkeit. Glasfasern sind jedoch steifer als ht-Fasern, so dass ein stabförmiger Körper, der sowohl ht-Fasern als auch Glasfasern umfasst, bevorzugt ist. Solch ein stabförmiger Körper kann optimal eingestellt werden hinsichtlich der Steifigkeit und der Flexibilität und im Hinblick auf die Torsionssteifigkeit.

Entsprechend einem weiteren Aspekt umfasst ein stabförmiger Körper
- ein oder mehrere nicht-metallische Filamente und
- ein nicht-ferromagnetisches Matrixmaterial,
dadurch gekennzeichnet, dass das Matrixmaterial die Filamente umschließt und/oder miteinander verklebt,
und Markerpartikel, die in der Röntgen- oder Magnetresonanzbildgebung ein Signal erzeugen. Dieser stabförmige Körper ist charakterisiert dadurch, dass ein oder mehrere nicht-metallische Filamente sich über den größten Teil der Länge des stabförmigen Körpers erstrecken.

Solche langen Filamente verleihen den stabförmigen Körpern eine hohe Steifigkeit in Längsrichtung.

Die nicht-metallischen Filamente sind elektrisch nicht leitfähige Filamente, so dass sie während MRT Messungen eingesetzt werden können. Folglich schließt der Begriff "nicht-metallische Filamente", wie er im vorliegenden Text verwendet wird, elektrisch leitfähige Filamente wie dünne Metalldrähte oder Carbonfasern aus.

Vorteilhafterweise bilden die Filamente einen Roving aus, der aus mehreren Filamenten, die parallel zueinander angeordnet sind, besteht.

Es ist jedoch auch möglich, dass die Filamente eines stabförmigen Körpers ein Garn ausbilden, was heißt, dass die Filamente miteinander verdrillt und/oder verflochten sind.

Entsprechend einem weiteren Aspekt umfasst ein medizinisches Instrument einen oder mehrere stabförmige Körper, umfassend
- ein oder mehrere nicht-metallische Filamente und
- ein nicht-ferromagnetisches Matrixmaterial,
dadurch gekennzeichnet, dass das Matrixmaterial die Filamente umschließt und/oder miteinander verklebt,
und Markerpartikel, die in der Röntgen- oder Magnetresonanzbildgebung ein Signal erzeugen, und ein Hüllpolymer, in welches der oder die stabförmigen Körper eingebettet sind, wobei ein Faden entweder in das Matrixmaterial oder in das Hüllpolymer eingebettet ist, dadurch charakterisiert, dass der Faden flexibler ist als das nicht-metallische Filament.

Die Steifigkeit des medizinischen Instruments und folglich der stabförmigen Körper in seitlicher Richtung muss in einem bestimmten Bereich liegen, der eine leichte Führbarkeit des medizinischen Instruments durch einen vorhandenen Hohlraum des menschlichen oder tierischen Körpers, z.B. in einem Blutgefäss, ermöglicht. Daher ist die Seitensteifigkeit begrenzt und es kann unter extremen Bedingungen vorkommen, dass das/die nicht-metallischen Filament(e) in dem/den stabförmigen Körper(n) brechen. In solch einem Fall sind die gebrochenen Teile des/der stabförmigen Körper(s) weiterhin durch den Faden miteinander verbunden, wobei zusätzlich das Hüllpolymer intakt bleibt. Das medizinische Instrument kann weiterhin sicher als ein Teil aus dem Körperhohlraum ohne das Risiko des Verlustes von Teilen im Blutstrom oder im Körpergewebe entfernt werden. Daher stellt der Faden ein Mittel zur Erhöhung der Sicherheit des medizinischen Instruments dar.

Der Faden ist vorzugsweise ein dünner Faden mit einer hohen Zugfestigkeit. Geeignete Fäden sind z.B. Polyamid-Fäden, ht-Fasern, Polyethylenterephthalat (PET) - Fasern, Kunstseide-Fasern (z.B. HL-Faser), Baumwoll-Fasern, oder Hanf-Fasern, welche vorzugsweise einen Durchmesser von 0,05 mm bis 0,2 mm haben. Falls der Faden eine oder mehrere ht-Fasern umfasst, können diese ht-Fasern gleichzeitig als die nicht-metallischen Filamente des stabförmigen Körpers fungieren. Selbstverständlich ist es möglich, den Faden in dem medizinischen Instrument unabhängig von den stabförmigen Körpern des medizinischen Instruments vorzusehen.

Entsprechend einem weiteren Aspekt umfasst ein medizinisches Instrument mehrere stabförmige Körper, umfassend
- ein oder mehrere nicht-metallische Filamente und
- ein nicht-ferromagnetisches Matrixmaterial,
dadurch gekennzeichnet, dass das Matrixmaterial die Filamente umschließt und/oder miteinander verklebt,
und ein Hüllpolymer, in welches die stabförmigen Körper eingebettet sind,
wobei die stabförmigen Körper in verschiedenen Positionen in Bezug auf den Mittelpunkt des medizinischen Instruments angeordnet sind und die stabförmigen Körper, die näher am Mittelpunkt des medizinischen Instruments angeordnet sind, nicht-metallische Filamente umfassen, die einen höheren Zugfestigkeitsmodul aufweisen als die nicht-metallischen Filamente der stabförmigen Körper, die weiter entfernt vom Mittelpunkt des medizinischen Instruments angeordnet sind.

Solch ein medizinisches Instrument umfassend nicht-metallische Filamente mit einer höheren Steifigkeit in den stabförmigen Körpern in seinem zentralen Abschnitt als in den nicht-metallischen Filamenten der stabförmigen Körper im äußeren Abschnitt kombiniert eine hohe Flexibilität mit einer hohen Steifigkeit.

Entsprechend einem weiteren Aspekt umfasst das medizinische Instrument einen langgestreckten Körper, z.B. einen Führungsdraht, einen Katheter oder einen Schlauch, bestehend aus einem Polymermaterial, und das Polymermaterial umschließt einen passiv-negativen MRT-Marker bestehend aus Markerpartikeln, die in der Magnetresonanztomographie ein Artefakt erzeugen, wobei der passiv-negative MRT-Marker nur in einem zentralen Abschnitt des medizinischen Instruments angeordnet ist.

Da der Marker im zentralen Abschnitt angeordnet ist, ist er von einem umlaufenden Abschnitt bedeckt, der keinen MRT-Marker beinhaltet. Daher besteht ein gewisser Abstand zwischen dem MRT-Marker und der äußeren Oberfläche des medizinischen Instruments. Im Gebrauch wird dieser Abstand des MRT-Markers von den das medizinische Instrument umgebenden Wassermolekülen beibehalten. Je größer dieser Abstand ist, desto kleiner werden die Artefakte im MRT-Bildgebungsprozess.

Der Abstand des passiv-negativen MRT-Markers zu der äußeren Oberfläche des medizinischen Instruments beträgt vorzugsweise mind. 0,1 mm, noch mehr bevorzugt mind. 0,2 mm, oder mind. 0,3 mm.

Solch ein medizinisches Instrument kann nicht-metallische Filamente umfassen und das genannte Polymermaterial bildet ein nicht-ferromagnetisches Matrixmaterial aus, welches die Filamente umschließt und/oder miteinander verklebt.

Solch ein medizinisches Instrument kann auch die oben beschriebenen stabförmigen Körper, welche den genannten MRT-Marker beinhalten, umfassen.

Solch ein medizinisches Instrument kann ein Führungsdraht sein, welcher einen stabförmigen Körper mit einem passiv-negativen MRT-Marker umfasst, der im Zentrum des Führungsdrahts angeordnet ist.

Solch ein Instrument kann auch ein Katheter oder ein Schlauch sein, der entweder zumindest einen stabförmigen Körper mit einem passiv-negativen MRT-Marker umfasst, wobei der stabförmige Körper am inneren Abschnitt des Katheters oder Schlauchs angeordnet ist, oder als zumindest zwei konzentrische Schichten ausgeführt wird, wobei nur die innerste Schicht einen passiv-negativen MRT-Marker enthält.

Wenn das medizinische Instrument als solch ein Katheter oder Schlauch mit zumindest zwei konzentrischen Schichten ausgeführt wird, kann eine der Schichten mit nicht-metallischen Filamenten, die zu einer räumlichen Struktur miteinander verzwirbelt, verflochten oder verwoben sind, versehen werden. Solch eine räumliche Struktur ist insbesondere bevorzugt in Kombination mit ht-Fasern. ht-Fasern sind flexibel und habe eine hohe Zugfestigkeit. Da in solch einer räumlichen Struktur die Filamente in verschiedenen Richtungen des Körpers des medizinischen Instruments laufen, bewirkt die hohe Zugfestigkeit auch eine hohe Steifigkeit des Verbundmaterials bestehend aus den Fasern und dem Matrixmaterial.

Entsprechend einem weiteren Aspekt der vorliegenden Erfindung umfasst ein medizinisches Instrument
- mehrere stabförmige Körper zur Verstärkung des medizinischen Instruments, und
- ein Hüllpolymer in welches die stabförmigen Körper eingebettet sind,
charakterisiert dadurch, dass das medizinische Instrument Markerpartikel umfasst, die in einem MRT-Prozess ein Artefakt erzeugen, und wobei das Hüllpolymer ein weiches Polymer oder Gummimaterial oder PVC ist.

Die stabförmigen Körper entsprechend diesem Aspekt der Erfindung können entsprechend den anderen Aspekten der vorliegenden Erfindung ausgeführt werden und/oder die nicht-metallischen verstärkenden Filamente können Glasfasern sein,

Die Marker können in die stabförmigen Körper und/oder das Hüllpolymer eingebettet sein.

Das spezifische Hüllpolymer entsprechend diesem weiteren Aspekt der vorliegenden Erfindung hat eine Relaxationszeit, die wesentlich kürzer ist als die von Wasser, aber deutlich länger als die eines harten Polymers wie z.B. Epoxidharz. Daher kann dieses Hüllpolymer, anders als harte Polymere, mit geeigneten Parametereinstellungen und einer kurzen Echozeit (vorzugsweise <100 ms, noch mehr bevorzugt <50 ms, und nochmals mehr bevorzugt <10 ms, am meisten bevorzugt <1 ms) in einem MRT-Prozess visualisiert werden. Insbesondere können die Protonen dieses Hüllpolymers mit einer MRT-Echozeit detektiert werden, die sich von derjenigen für die Detektion von Wasserprotonen unterscheidet. Daher können mit der Benutzung von zwei unterschiedlichen Echozeiten gleichzeitig zwei verschiedene Bilder des gleichen Objekts mit der gleichen Darstellung aufgenommen werden, wobei das eine Bild klar das medizinische Instrument darstellt (durch Messung der Relaxation der Protonen im Hüllpolymer) und das andere Bild das Körpergewebe (durch Messung der Relaxation der Protonen im Wasser und im Fett, welches im Körpergewebe oder im Blut enthalten ist). Beide Bilder können überlagert werden, so dass der Arzt die kombinierte Information in einem Bild erhält. Durch die Detektion der Protonen im weichen Polymer und nicht in den Wassermolekülen, die das medizinische Instrument umgeben, kann ein begrenzteres und wesentlich schärferes Artefakt erhalten werden, welches fast auf den tatsächlichen Durchmesser des medizinischen Instruments begrenzt ist.

Dieses Hüllpolymer hat vorzugsweise eine T1 Relaxationszeit von 1 bis 100 ms, noch mehr bevorzugt von 1 bis 500 ms, and am meisten bevorzugt von 1 bis 1000 ms, und eine T2 Relaxationszeit von vorzugsweise 0,1 is 1 ms, noch mehr bevorzugt von 0,1 bis 5ms und am meisten bevorzugt von 0,1 bis 10 ms.

In einer weiteren Ausführungsform der vorliegenden Erfindung hat das medizinische Instrument eine stabil kovalent an seine äußere Oberfläche angebundene Beschichtung. Diese Beschichtung ist vorzugsweise gleitfähig. Das stabil angebundene Beschichtungsmaterial wird durch die Mischung eines Hüllpolymers mit einer oder mehreren chemischen Substanzen mit freien funktionellen Gruppen, vorzugsweise Carboxylgruppen oder Aminogruppen, erhalten. Die Einbettung der stabförmigen Körper in dieses modifizierte Hüllpolymer wird vorzugsweise durch einen Extrusionsprozess erreicht. Nachfolgend werden die funktionellen Gruppen auf der Oberfläche, vorzugsweise die Carboxyl/Aminogruppen, mit anderen funktionellen Gruppen, vorzugsweise mit Amino/Carboxylgruppen, reagiert, um eine kovalente Bindung zu erhalten, vorzugsweise eine Amidbindung. Die verbleibenden funktionellen Gruppen (z.B. die verbleibenden Carboxyl/Aminogruppen) werden dann chemisch mit einem Quervernetzer quervernetzt.

Die vorstehend beschriebenen verschiedenen Aspekte der Erfindung können miteinander kombiniert werden.

Die Erfindung wird im Folgenden anhand der Zeichnungen näher erläutert. Diese zeigen in:

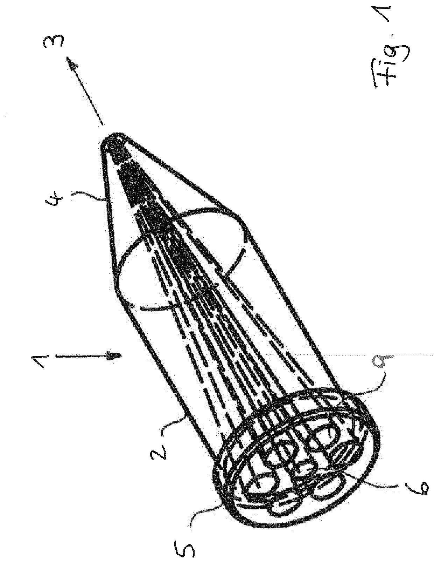

- Fig. 1: eine schematische Darstellung einer erfindungsgemäßen Pinole zum Extrudieren eines strukturierten Extrudats, wobei der Pinolenkörper transparent dargestellt ist, sodass Führungskanäle sichtbar sind,

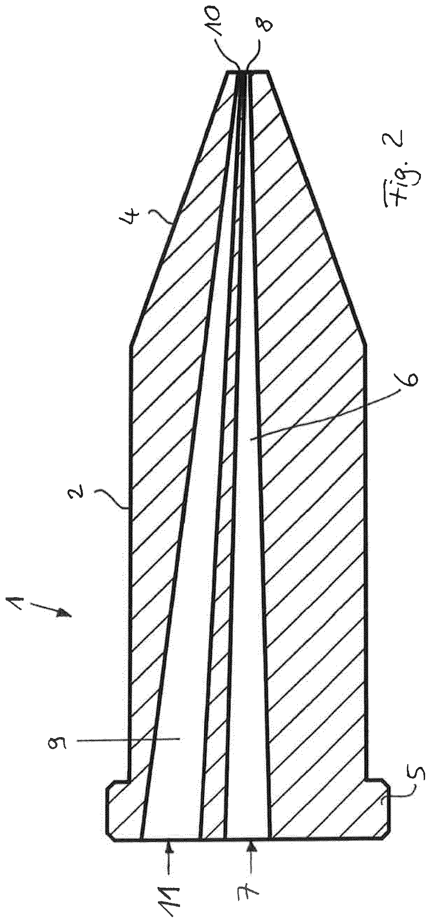

- Fig. 2: eine seitlich geschnittene Ansicht einer erfindungsgemäßen Pinole mit einem zentralen Führungskanal und einem peripheren Führungskanal,

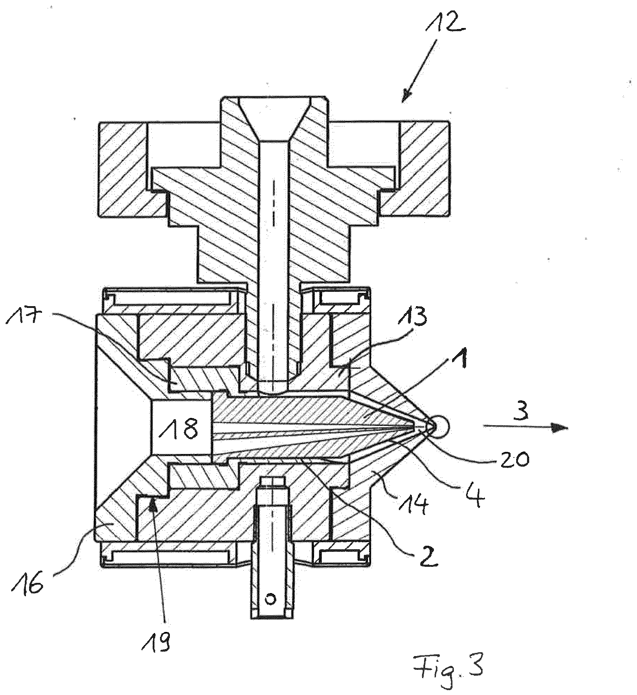

- Fig. 3: eine schematische Darstellung einer erfindungsgemäßen Vorrichtung mit der erfindungsgemäßen Pinole,

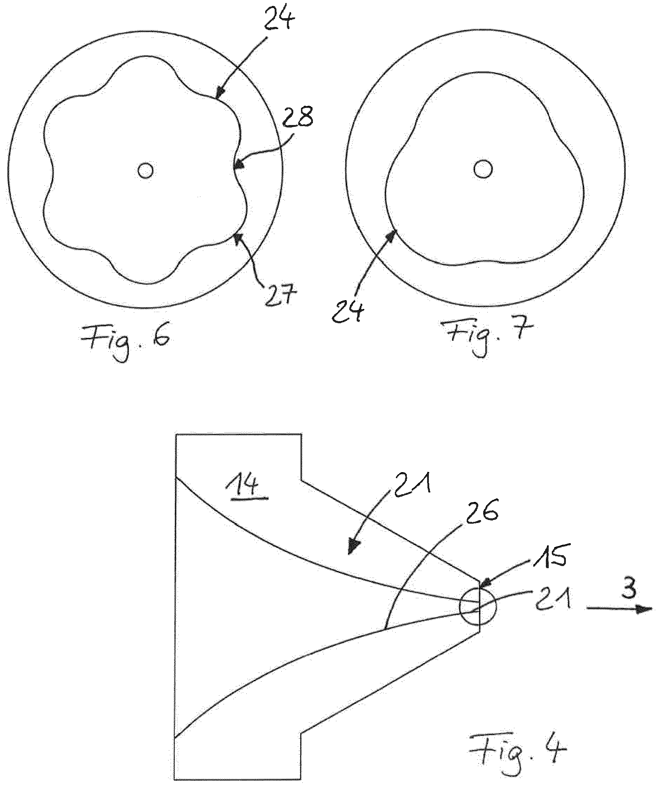

- Fig. 4: eine schematische Darstellung einer erfindungsgemäßen Düsenwandung in einer seitlich geschnittenen Ansicht,

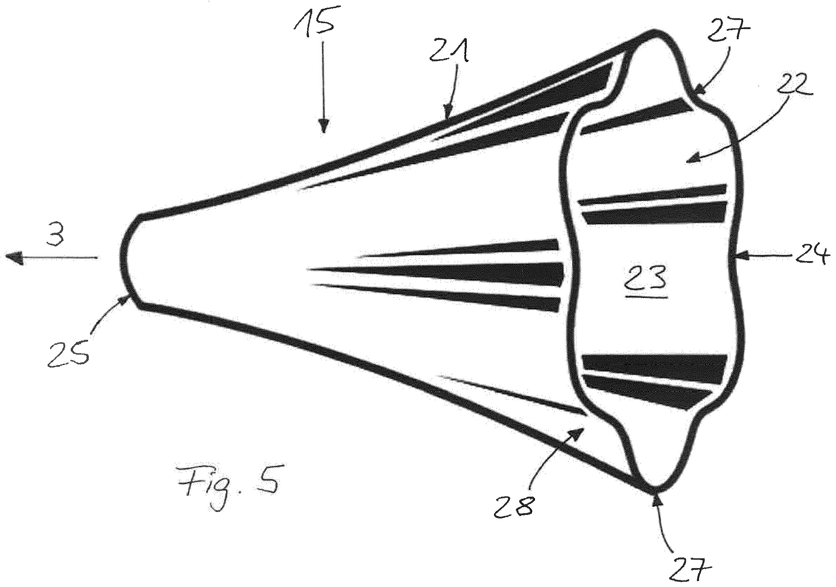

- Fig. 5: eine perspektivische Ansicht eines erfindungsgemäßen Schmelzekanals einer Austrittsdüse,
- Fig. 6: eine Eintrittsöffnung des Schmelzekanals in einer Draufsicht,
- Fig. 7: eine alternative Ausführungsform einer Eintrittsöffnung des Schmelzekanals in einer Draufsicht.

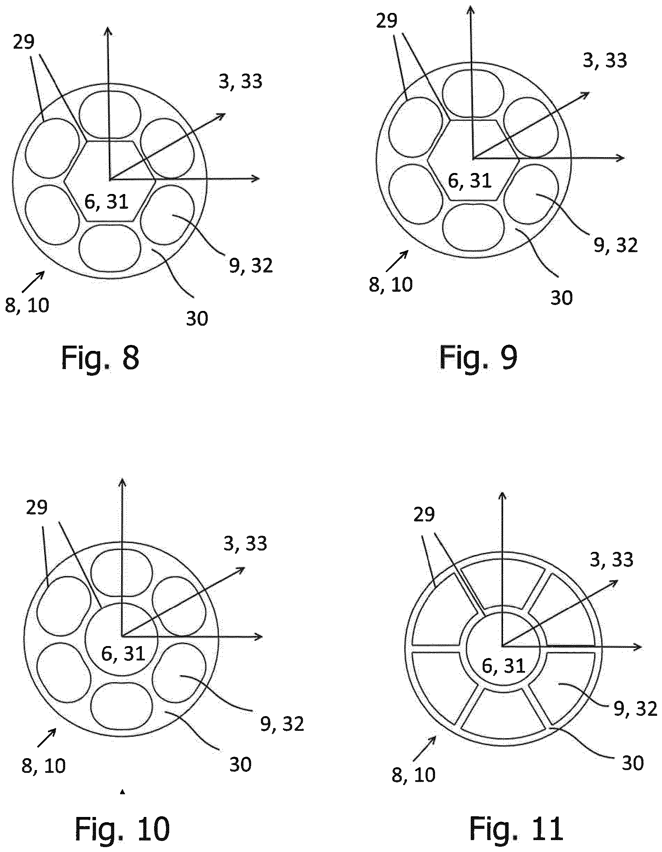

- Figur 8: eine schematische Darstellung eines Querschnitts quer zur Produktionsrichtung einer erfindungsgemäßen Pinole im Bereich einer Ausrichtöffnung mit einem zentralen Führungskanal und sechs peripheren Führungskanälen bzw. eine schematische Darstellung eines medizinischen Instruments mit einer der Anzahl der Führungskanäle entsprechenden Anzahl an stabförmigen Körpern,
- Figur 9: eine weitere Ausführungsform eines Querschnitts quer zur Produktionsrichtung einer erfindungsgemäßen Pinole im Bereich einer Ausrichtöffnung mit einem zentralen vieleckigen Führungskanal und sechs elliptischen peripheren Führungskanälen in einer schematischen Darstellung bzw. eine schematische Darstellung eines medizinischen Instruments mit einer der Anzahl der Führungskanäle entsprechenden Anzahl an stabförmigen Körpern,
- Figur 10: eine weitere Ausführungsform eines Querschnitts quer zur Produktionsrichtung einer erfindungsgemäßen Pinole im Bereich einer Ausrichtöffnung mit einem zentralen vieleckigen Führungskanal und sechs peripheren elliptischen Führungskanälen in einer schematischen Darstellung bzw. eine schematische Darstellung eines medizinischen Instruments mit einer der Anzahl der Führungskanäle entsprechenden Anzahl an stabförmigen Körpern,
- Figur 11: eine weitere Ausführungsform eines Querschnitts quer zur Produktionsrichtung einer erfindungsgemäßen Pinole im Bereich einer Ausrichtöffnung mit einem zentralen runden Führungskanal und sechs peripheren trapezförmigen Führungskanälen in einer schematischen Darstellung bzw. eine schematische Darstellung eines medizinischen Instruments mit einer der Anzahl der Führungskanäle entsprechenden Anzahl an stabförmigen Körpern,

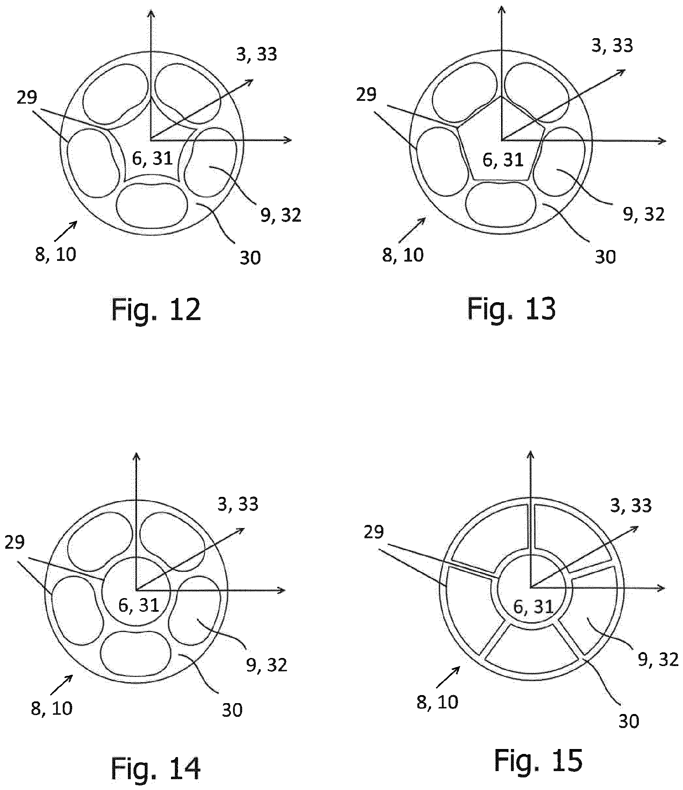

- Figur 12: eine weitere Ausführungsform eines Querschnitt quer zur Produktionsrichtung einer erfindungsgemäßen Pinole im Bereich einer Ausrichtöffnung mit einem zentralen trapezförmigen Führungskanal und sechs peripheren elliptischen Führungskanälen in einer schematischen Darstellung bzw. eine schematische Darstellung eines medizinischen Instruments mit einer der Anzahl der Führungskanäle entsprechenden Anzahl an stabförmigen Körpern,
- Figur 13: eine weitere Ausführungsform eines Querschnitts quer zur Produktionsrichtung einer erfindungsgemäßen Pinole im Bereich einer Ausrichtöffnung mit einem zentralen Führungskanal und sechs peripheren Führungskanälen in einer schematischen Darstellung bzw. eine schematische Darstellung eines medizinischen Instruments mit einer der Anzahl der Führungskanäle entsprechenden Anzahl an stabförmigen Körpern,
- Figur 14: eine weitere Ausführungsform eines Querschnitts quer zur Produktionsrichtung einer erfindungsgemäßen Pinole im Bereich einer Ausrichtöffnung mit einem zentralen runden Führungskanal und sechs peripheren elliptisch Führungskanälen in einer schematischen Darstellung bzw. eine schematische Darstellung eines medizinischen Instruments mit einer der Anzahl der Führungskanäle entsprechenden Anzahl an stabförmigen Körpern, und
- Figur 15: eine weitere Ausführungsform eines Querschnitts quer zur Produktionsrichtung einer erfindungsgemäßen Pinole im Bereich einer Ausrichtöffnung mit einem zentralen runden Führungskanal und sechs peripheren trapezförmig Führungskanälen in einer schematischen Darstellung bzw. eine schematische Darstellung eines medizinischen Instruments mit einer der Anzahl der Führungskanäle entsprechenden Anzahl an stabförmigen Körpern.

Erfindungsgemäß ist eine Vorrichtung 12 zum Extrudieren eines strukturierten Extrudats, insbesondere eines medizinischen Instruments, das in einen menschlichen oder tierischen Körper einführbar ist, vorgesehen (Figur 3).

Die Vorrichtung 12 umfasst ein Gehäuse, wobei das Gehäuse aus einer umlaufenden Seitenwandung 13, das an einem in Produktionsrichtung 3 vorne liegenden Ende mit einer Düsenwandung 14 mit einer Austrittsdüse 15 und an einem in Produktionsrichtung 3 hinten liegenden Ende mit einem Fixierring 16, einem Hülsenelement 17 und einer Pinole 1 versehen ist.

Der Fixierring 16 ist ringartig ausgebildet und umfasst in Produktionsrichtung 3 eine Durchgangsöffnung 18 die von einem in Produktionsrichtung 3 sich konisch verjüngenden Abschnitt in einen zylindrischen Abschnitt übergeht. Außenseitig bzw. radial umlaufend weist der Fixierring zwei umlaufende Stufen 19 auf.

Über die Durchgangsöffnung 18 gelangen die stabförmigen Körper zur Pinole 1 wobei sie dabei die Wandung der Durchgangsöffnung nicht kontaktieren.

In Produktionsrichtung 3 ist vor dem Fixierring 16 das rohrartig ausgebildete Hülsenelement 17 angeordnet.

Ein an der Pinole 1 angeformter Ringabschnitt 5 wird im Bereich zwischen dem Hülsenelement 17 und dem Fixierring 16 fixiert. Die Fixierung der Pinole 1 erfolgt, indem der Fixierring 16 mit der Seitenwandung 13 verschraubt wird. Auf diese Weise wird der Ringabschnitt 28 der Pinole 1 zwischen Fixierring 16 und Hülsenelement eingeklemmt.

Vorzugsweise ist ein Mittel, wie z.B. ein Passstift, an der Pinole 1 vorgesehen, um eine Selbstausrichtung der Pinole in einer Winkelstellung bzgl. der Produktionsrichtung in entsprechender Flucht zur Düsenwandung zu gewährleisten.

Der Passstift kann in eine entsprechende in dem Fixierring ausgebildete Ausnehmung eingreifen.

Alternativ kann die Pinole um die Produktionsrichtung drehbar bzw. in ihrer Winkelstellung bzgl. der Produktionsrichtung frei oder auch in Rastern mit einem Winkelversatz von 1° bis 60° bzw. 2° bzw. 5° bzw. 10° bzw. 15° bzw. 30° bzw. 45° einstellbar sein. Wenn dann der Fixierring 16 und die Seitenwandung 13 miteinander verbunden sind, bspw. mittels einer Schraubenverbindung, ist die Pinole ortsfest im Gehäuse angeordnet.

Eine Stirnseite des Hülsenelements 17 ist derart konkav abgeschrägt, dass die Schmelze in Produktionsrichtung zwischen Seitenwandung 13 und Pinole strömt. Die Stirnseite des Hülsenelements 17 begrenzt den Extrusionsraum entgegen der Produktionsrichtung 3.

An ihrer in Produktionsrichtung vorne liegenden Stirnwandung ist die Seitenwandung mit der Düsenwandung, bspw. mittels einer Schraubenverbindung, verbunden. Hierbei kann auch vorgesehen sein, dass die Düsenwandung um die Produktionsrichtung drehbar bzw. in ihrer Winkelstellung bzgl. der Produktionsrichtung frei oder auch in gerastert mit einem Winkelversatz von 1° bis 60° bzw. 2° bzw. 5° bzw. 10° bzw. 15° bzw. 30° bzw. 45° einstellbar ist.

Im Folgenden wird die Pinole 1 beschrieben. Die Pinole 1 weist einen zylindrischen Abschnitt 2 und einen sich in Produktionsrichtung 3 daran anschließenden kegelstumpfförmigen Abschnitt 4 auf (Figur 1 und Figur 2).

An einem in Produktionsrichtung 3 hinten liegendem Ende der Pinole 1 ist der als Stufe ausgebildete Ringabschnitt 5 angeformt, mittels dem die Pinole im Gehäuse fixierbar ist.

In der Pinole 1 ist axial fluchtend zur Produktionsrichtung 3 ein konisch ausgebildeter zentraler Führungskanal 6 vorgesehen.

Der zentrale Führungskanal 6 weist eine im Wesentlichen konstante Konizität auf.

An einem in Produktionsrichtung 3 hinten liegenden Ende des zentralen Führungskanals 6 werden die Kanalöffnungen als Zuführöffnungen 7 bezeichnet. Die in Produktionsrichtung vorne liegenden Öffnungen des zentralen Führungskanals 6 werden als Ausrichtöffnungen 8 bezeichnet.

Die in Produktionsrichtung vorne liegende Öffnung des zentralen Führungskanals 6 bzw. die Ausrichtöffnung 8 weist einen Durchmesser von 0,2 mm bis 0,4 mm und insbesondere von 0,3 mm auf.

Die in Produktionsrichtung 3 hinten liegende Öffnung bzw. die Zuführöffnung 7 des zentralen Führungskanals 6 weist einen Durchmesser von 2,0 mm bis 4,0 mm und insbesondere von 3,0 mm auf.

Weiterhin ist in der Pinole 1 zumindest ein neben dem zentralen Führungskanal 6 angeordneter peripherer Führungskanal 9 ausgebildet. Der periphere Führungskanal 9 weist ebenfalls eine konstante Konizität über seine gesamte Länge auf.

Eine in Produktionsrichtung 3 vorne liegende Öffnung des peripheren Führungskanals 9 wird als periphere Ausrichtöffnung 10 bezeichnet und weist einen Durchmesser von 0,1 mm bis 0,3 mm und insbesondere von 0,2 mm auf.

Eine in Produktionsrichtung 3 hinten liegende Öffnung des peripheren Führungskanals 9 wird als periphere Zuführöffnung 11 bezeichnet und weist einen Durchmesser von 3,0 mm bis 5,0 mm und insbesondere von 4,0 mm auf.

Der periphere Führungskanal 9 ist gegenüber der Produktionsrichtung 3 in einem Winkel von 0° bis 30° bzw. von 2,5° bis 15° und insbesondere von 5° bis 10° geneigt. Diese Neigung bezieht sich auf eine in Produktionsrichtung verlaufende Mittelachse des konischen peripheren Führungskanals.

Wenn mehr als 6 periphere Rods zugeführt werden sollen können auch steilere Winkel bzgl. der Produktionsrichtung 3 vorgesehen werden, um die entgegen der Produktionsrichtung 3 liegenden Öffnungen 11 der peripheren Führungskanäle 9 entsprechend anordnen zu können. Ggfs. kann der Durchmesser dieser Öffnungen 11 reduziert werden. Es kann auch vorgesehen sein weitere Parameter der Vorrichtung wie z.B. Durchmesser des Führungskanals, Durchmesser der Pinole, Durchmesser eines Verteilungskreises der Öffnungen 11 der Führungskanäle 9 sowohl in als auch entgegen der Produktionsrichtung 3 zu verändern.

Eine Innenfläche bzw. Wandung des zentralen Führungskanals 6 und des peripheren Führungskanals 9 weist eine Oberflächenrauheit R_{A} kleiner gleich 1,0 µm auf.

Gemäß einer weiteren Ausführungsform der erfindungsgemäßen Pinole 1 (Fig. 1, Fig. 2) sind in der Pinole 1 ein zentraler Führungskanal 6 und drei bzw. sechs den zentralen Führungskanal 6 konzentrisch umgebende und radial gleich beabstandet voneinander angeordnete periphere Führungskanäle 9 vorgesehen. Die peripheren Führungskanäle 9 sind bezüglich der Produktionsrichtung 3 bzw. bezüglich dem zentralen Führungskanal 6 in einem Abstand von 60° zueinander angeordnet.

Die Pinole 1 ist von der Seitenwandung 5 umgeben, wobei eine in der Seitenwandung 5 ausgebildete Stufe an einer Stirnwandung des Hülsenelements 17 anliegt.

Im Folgenden werden weitere Bauteile der Vorrichtung beschrieben (Figur 3 bis Figur 7).

Am in Produktionsrichtung 3 vorne liegenden Ende ist die Düsenwandung 14 mit der Seitenwandung 13 verbunden. Die Verbindung kann derart erfolgen, dass die Düsenwandung in ihrer Winkelstellung bzgl. der Produktionsrichtung 3 in vorbestimmten Intervallen oder sogar frei einstellbar ist.

Der Raum zwischen der Pinole 1, dem Hülsenelement 17, der Seitenwandung 13 und der Düsenwandung 14 bildet den Extrusionsraum 20 aus.

Die Düsenwandung 14 ist im Bereich des Extrusionsraumes 20 sich in Produktionsrichtung 3 konisch verjüngend und somit als ein ringförmiger Kegelstumpf 21 ausgebildet. Dieser Kegelstumpf weist eine bezüglich des Extrusionsraumes 20 konvex ausgebildete Mantelwandung auf.

Die Krümmung einer Mantelwandung des Kegelstumpfes 4 der Pinole entspricht vorzugsweise der Krümmung der Düsenwandung 14. Das heißt, dass diese korrespondierend ausgebildet ist, um einen konstanten Ringspalt zwischen Kegelstumpf 4 der Pinole und dem Kegelstumpf 26 der Düsenwandung 14 auszubilden. Demnach ist die Mantelwandung des Kegelstumpfes 4 der Pinole bzgl. des Extrusionsraumes konkav ausgebildet, um mit der konvexen Düsenwandung 14 bzw. deren Kegelstumpf 26 zu korrespondieren (konkav/konvex).

Der Abstand bzw. der Ringspalt zwischen dem kegelförmigen Abschnitt 4 der Pinole 1 und dem Kegelstumpf 26 der Düsenwandung beträgt 1,0 mm bis 10 mm bzw. 1,0 mm bis 5 mm bzw. 1,0 mm bis 3 mm bzw. 1,0 bis 2,0 mm.

Die konkave 4 und die konvexe Wandung 26 lassen sich somit bzgl. eines außerhalb der Vorrichtung 12 liegenden Mittelpunktes als konzentrische Kreise definieren. Der Durchmesser dieser Kreise liegt im Bereich zwischen 100 mm und 200 mm und vorzugsweise zwischen 130 mm und 160 mm.

In Alternativen nicht bevorzugten Ausführungsformen können die Wandungen des kegelförmigen Abschnitts 4, 26 der Pinole 1 und der Düsenwandung 14 auch eben/konvex, konvex/eben eben/eben, eben/konkav, konkav/eben konvex/konkav ausgebildet sein.

Die Austrittsdüse 15 umfasst einen Schmelzekanal 22, wobei der Schmelzekanal 22 sich in Produktionsrichtung 3 konisch verjüngend als ein ringförmiger Kegelstumpf 21 mit einer Anordnungswandung 23 ausgebildet ist. Eine entgegen der Produktionsrichtung 3 liegende Öffnung des Schmelzekanals 22 wird als Eintrittsöffnung 24 bezeichnet. Eine in Produktionsrichtung 3 liegende Öffnung des Schmelzekanals wird als Austrittsöffnung 25 bezeichnet.

Die Eintrittsöffnung 24 kann im Querschnitt insbesondere gewellt oder gezackt ausgebildet sein. Die sich daraus ergebenden, sich in Produktionsrichtung 3 erstreckenden Konturen werden als Rinnen 27 bezeichnet.

Die Austrittsöffnung 25 kann im Querschnitt insbesondere kreisförmig ausgebildet sein.

Ein Abschnitt der Austrittsöffnung kann in etwa über eine Länge von 1 mm zylindrisch ausgebildet sein, um ein geradliniges Auslaufen des Extrudats aus der Vorrichtung sicherzustellen.

Demgemäß sind in der Anordnungswandung 23 des Schmelzekanals 22 sich vom gewellten Querschnitt der Eintrittsöffnung 24 aus, sich in Produktionsrichtung 3 erstreckende Rinnen 27 ausgebildet, wobei die Rinnen 27 dann in den kreisförmigen Querschnitt der Austrittsöffnung 25 übergehen. Daher wird die Austrittsdüse 15 als strukturierte Austrittsdüse 15 bezeichnet.

Die Anzahl der konturierten Rinnen 27 kann vorzugsweise der Anzahl der peripheren Führungskanäle 9, 10, 11 entsprechen.

Vorzugsweise sind die Rinnen 17 um den halben Abstandswinkel der peripheren Führungskanäle 9, 10, 11 versetzt zu diesen angeordnet sein.

Die Kontur der Anordnungswandung 23 kann somit im Querschnitt dreiecksförmig mit Einschnürungen in den Seiten bzw. blütenförmig ausgebildet sein. Insbesondere entspricht die Anzahl der Blütenblätter der Anzahl der peripheren Führungskanäle 9.

Vorzugsweise ist die erfindungsgemäße Vorrichtung zum Herstellen von strukturierten Extrudaten mit zum Beispiel einem zentralen und zwei bis zehn und vorzugsweise drei bis sechs peripheren stabförmigen Körpern ausgebildet. Entsprechend der mechanischen Anforderung an das strukturierte Extrudat können ein zentraler stabförmiger Körper und mehrere periphere stabförmige Körper vorgesehen sein. Grundsätzlich ist eine beliebige Anordnung der stabförmigen Körper mit oder ohne einen zentralen stabförmigen Körper möglich.

Die erfindungsgemäße Vorrichtung kann auch zum Herstellen von Schläuchen oder Kathetern mit zum Beispiel drei bis zehn stabförmigen Körpern ausgebildet sein, die in der Schlauch- bzw. Katheterwandung angeordnet sind.

Ein System zum Extrudieren eines strukturierten Extrudats umfasst entlang einer Produktionsrichtung eine Einrichtung zum Zuführen stabförmiger Körper, wie z.B. ein Spulenbaum (nicht dargestellt), die Vorrichtung, eine Kühleinrichtung, wie z.B. ein Wasserbad (nicht dargestellt), eine Ausrichteinrichtung (nicht dargestellt) und eine Abzugseinrichtung (nicht dargestellt). Die Vorrichtung umfasst den Extrusionsraum 3, in den eine Schmelze- oder Polymerzuführeinrichtung mündet.

Bevorzugt ist zwischen der Kühleinrichtung und der Abzugseinrichtung eine Ausrichteinrichtung vorgesehen.

Im Folgenden werden verschiedene Ausführungsformen des zentralen Führungskanals 6 und der peripheren Führungskanäle 9 beschrieben. Der zentrale Führungskanal 6 und die peripheren Führungskanäle 9 können dabei einen konturierten Querschnitt aufweisen, der oval bzw. bohnenförmig oder drei- oder vier- oder vieleckig, trapezförmig oder elliptisch ausgebildet ist. Die dargestellten Ausschnitte sind schematische Darstellungen und betreffen einen Bereich der Pinole 1 in etwa im Bereich der Ausrichtöffnung 8. Ein mit einer solchen Pinole 1 hergestelltes strukturiertes Extrudat weist exakt eine solche Anordnung der stabförmigen Körper 29 im Querschnitt des strukturierten Extrudats 30 auf.

Gemäß einem weiteren Ausführungsbeispiel umfasst die Pinole 1 einen zentralen Führungskanal 6 mit sechseckigem Querschnitt, wobei die Kanten des Sechsecks bezüglich einer quer zur Produktionsrichtung 3 liegenden Ebene konvex ausgebildet sind (Figur 8). Im Bereich der konvexen Kanten des sechseckigen zentralen Führungskanals 6 sind radial umlaufend und gleich beabstandet voneinander jeweils ein elliptischer peripherer Führungskanal 9 derart angeordnet, dass insgesamt in der Pinole sechs periphere Führungskanäle 9 angeordnet sind.

Gemäß einer weiteren Ausführungsform der Pinole weist der zentrale Führungskanal 6 einen sechseckigen Querschnitt mit gleichen Kantenlängen auf, wobei im Bereich der Kanten des Sechsecks des zentralen Führungskanals 6 radial umlaufend und in etwa gleich beabstandet voneinander sechs periphere Führungskanäle 9 mit elliptischem Querschnitt vorgesehen sind (Figur 9).

Gemäß einer weiteren Ausführungsform weist der zentrale Führungskanal 6 einen runden Querschnitt auf, wobei ebenfalls radial gleich beabstandet voneinander sechs periphere Führungskanäle 9 mit elliptischem Querschnitt vorgesehen sind (Figur 10).

Gemäß einer weiteren Ausführungsform weist der zentrale Führungskanal einen runden Querschnitt auf, wobei die peripheren Führungskanäle 9 einen in etwa trapezförmigen Querschnitt aufweisen (Figur 11). Der trapezförmige Querschnitt der peripheren Führungskanäle 9 ist dabei derart ausgebildet, dass die in radialer Richtung bezüglich der Produktionsrichtung verlaufenden Kanten der Trapeze in etwa parallel zueinander angeordnet sind und die beiden in etwa parallel zueinander verlaufenden Kanten des Trapezes bezüglich der Produktionsrichtung in vertikaler Richtung konkav ausgebildet sind.

Gemäß einer weiteren Ausführungsform der Pinole umfasst diese einen zentralen Führungskanal 6 mit fünfeckigem Querschnitt, wobei die Kanten des Fünfecks bezüglich der Produktionsrichtung konkav ausgebildet sind (Figur 12). Ähnlich dem anhand von Figur 8 beschriebenen Ausführungsbeispiel sind fünf periphere Führungskanäle 9 vorgesehen, die im Bereich der Kanten des zentralen Führungskanals 6 angeordnet sind und wobei eine entlang der Kante des zentralen Führungskanals 6 ausgebildete Kante der peripheren Führungskanäle 9 bezüglich der Produktionsrichtung konvex ausgebildet ist (Figur 12).

Gemäß einer weiteren Ausführungsform kann der zentrale Führungskanal 6 als gleichseitiges Vieleck, insbesondere als Fünfeck, ausgebildet sein. Demgemäß sind entsprechend fünf periphere Führungskanäle mit elliptischem Querschnitt vorgesehen, deren Kanten bezüglich einer Ebene quer zur Produktionsrichtung 3 konvex ausgebildet sind (Figur 13).

Gemäß einer weiteren Ausführungsform der Pinole 1 ist der zentrale Führungskanal 6 rund ausgebildet, wobei radial umlaufend und gleich beabstandet voneinander fünf elliptische periphere Führungskanäle 9 vorgesehen sind, deren Kanten bezüglich einer Ebene quer zur Produktionsrichtung 3 bezüglich der Produktionsrichtung 3 konvex ausgebildet ist (Figur 14).

Gemäß einer weiteren Ausführungsform, die im Wesentlichen der anhand von Figur 11 beschriebenen Ausführungsform entspricht, können anstelle von sechs peripheren Führungskanälen auch fünf radial gleich beabstandet voneinander angeordnete und in etwa trapezförmig ausgebildete periphere Führungskanäle 9 vorgesehen sein (Figur 15).

Eine erfindungsgemäße Pinole 1 umfasst in der Regel einen zentralen Führungskanal 6 dessen Querschnitt vorzugsweise rund oder vieleckig ausgebildet ist, wobei die Kanten des Vielecks bezüglich einer Ebene quer zur Produktionsrichtung 3 konvex oder konkav ausgebildet sein können.

Weiterhin umfasst eine solche Pinole 1 vorzugsweise zumindest drei oder vier oder fünf oder sechs oder sieben oder acht oder neun oder mehr periphere Führungskanäle 9, die elliptisch, vieleckig, trapezförmig ausgebildet sein können, und wobei deren Kanten bezüglich der Produktionsrichtung konkav oder konvex geformt sind.

Im Bereich der Zuführöffnungen 7, 11 können die radialen Abstände der peripheren Führungskanäle 9 zueinander größer bzw. weiter sein. Gleiches gilt für die Abstände der peripheren Führungskanäle 9 zum zentralen Führungskanal 6.

Im Folgenden wird ein erfindungsgemäßes Verfahren zum Herstellen von Führungsdrähten oder Kathetern zum Einbringen in den menschlichen oder tierischen Körper beispielhaft anhand eines Führungsdrahtes, in dem sieben stabförmige Körper angeordnet sind, beschrieben.

Gemäß dem erfindungsgemäßen Verfahren werden bspw. sieben stabförmige Körper von jeweils auf einer Einrichtung zum Zuführen stabförmiger Körper angeordneten Spulen durch entsprechende Zuführabschnitte und Ausrichtabschnitte des Zuführelements hinein in die Führungskanäle geführt.

Die sieben stabförmigen Körper werden durch die Führungskanäle in Produktionsrichtung durch die Pinole hindurch dem Extrusionsraum zugeführt. In den Führungskanälen bzw. deren Ausrichtöffnungen 8, 10 erfolgt die exakte Anordnung der stabförmigen Körper.

Dem Extrusionsraum wird über eine seitlich angeordnete Polymer-Zuführeinrichtung eine Polymermasse in fließfähigem Zustand zugeführt.

Das Gehäuse wird mittels einer Heizeinrichtung beheizt, um die Polymermasse in fließfähigem Zustand zu halten.

Die einzelnen stabförmigen Körper werden beim Austritt aus den in Produktionsrichtung liegenden Enden der Führungskanäle mit Schmelze beaufschlagt. Die Zwischenräume der einzelnen stabförmigen Körper werden dadurch mit Polymer aus dem Extrusionsraum aufgefüllt, wodurch diese in das Polymer eingebettet bzw. zusammengeklebt werden.

Beim nachfolgenden Hindurchführen der stabförmigen Körper durch den Schmelzekanal der Austrittsdüse kommt es aufgrund der Relativbewegung zwischen der Schmelze und den stabförmigen Körpern zu einer optimalen Benetzung der stabförmigen Körper mit Schmelze. Weiterhin wird durch den Schmelzekanal und dessen geradlinig fluchtende Anordnung zu den Führungskanälen die Anordnung der stabförmigen Körper zueinander stabilisiert und erhalten.

Erfindungsgemäß ist vorgesehen, dass die stabförmigen Körper beim Eintritt in den Schmelzekanal 22 im Bereich der Einbuchtungen 28 angeordnet sind. Eine derartige Anordnung bewirkt, dass die stabförmigen Körper im Extrudat mit ausreichend Polymer beaufschlagt sind bzw. dass die stabförmigen Körper nicht aus dem Extrudat herausragen.

Das bedeutet, dass die Ausbuchtungen bzw. die Rinnen 27 um den halben Abstandswinkel der peripheren Führungskanäle versetzt zu diesen angeordnet sind.

Demgemäß sind die peripheren Führungskanäle 9 der Pinole 1 in etwa axial fluchtend zu den Einbuchtungen 28 des Schmelzekanals 22 angeordnet. Eine derartige Ausrichtung der peripheren Führungskanäle zu den Einbuchtungen des Schmelzekanals bewirkt, dass das in den Ausbuchtungen 27 strömende Polymer im Schmelzekanal 22 beim Übergang von dem konturierten Abschnitt hin zur runden Austrittsöffnung 25 in den Bereich gedrückt wird in dem die stabförmigen Körber angeordnet sind, so dass diese bei Austritt aus der Vorrichtung ausreichend mit Polymer überdeckt sind und ein rundes Extrudat bereitstellbar ist.

Weiterhin wird durch diesen Versatz ein geringfügiger Druck auf die stabförmigen Körper ausgeübt, so dass diese die exakte geometrische Anordnung im strukturierten Extrudat einnehmen können.

Wenn jedoch die stabförmigen Körper im Extrudat weiter außen angeordnet werden sollen kann vorgesehen sein, die peripheren Führungskanäle 9 der Pinole 1 in etwa axial fluchtend zu den Ausbuchtungen bzw. den Rinnen 27 des Schmelzekanals angeordnet. Eine derartige Ausrichtung der peripheren Führungskanäle zu den Ausbuchtungen des Schmelzekanals bewirkt, dass das in den Ausbuchtungen 28 strömende Polymer im Schmelzekanal beim Übergang von dem konturierten Abschnitt hin zur runden Austrittsöffnung in den Bereich zwischen den stabförmigen Körper gedrückt wird, sodass diese beim Austritt aus der Vorrichtung ein rundes Extrudat bereitstellbar ist.

Sollte der Abstand der stabförmigen Körper in den Führungskanälen größer sein als der Abstand, den sie im strukturierten Extrudat aufweisen sollen, ist vorgesehen, dass der Durchmesser des Schmelzekanals derart ausgebildet ist, dass die stabförmigen Körper beim Austritt aus der Austrittsdüse durch den Schmelzekanal, insbesondere durch die Einbuchtungen zusammengedrückt werden. Das Zusammendrücken der stabförmigen Körper kann insbesondere dann erforderlich sein, wenn der Abstand der einzelnen stabförmigen Körper zueinander geringer sein soll als der aufsummierte Wandabstand der Führungskanäle.

Von Bedeutung für diesen Produktionsschritt ist, dass der Abstand von den in Produktionsrichtung vorne liegenden Enden der Führungskanäle bis zur Austrittsdüse nicht zu lang, insbesondere maximal 8 mm ist, da dieser bestimmt, wie lange die stabförmigen Körper mit Schmelze beaufschlagt werden.

Beim Austritt aus der Austrittsdüse erfolgt dann die endgültige Festlegung der Geometrie des strukturierten Extrudats, die durch den Durchmesser des Schmelzekanals und insbesondere der Austrittsöffnung 25 der Austrittsdüse festgelegt wird.

Anschließend wird der auf diese Weise erzeugte Führungsdraht in einem Wasserbad abgekühlt.

Eine Ausrichteinrichtung gewährleistet eine geradlinig fluchtende axiale Ausrichtung des strukturierten Extrudats zum Schmelzekanal der Austrittsdüse und den Führungskanälen derart, dass diese durch die Polymerschmelze möglichst wenig beeinflusst werden und gleichmäßig mit Polymer ummantelt werden können.

Das Hindurchführen der stabförmigen Körper durch die Extrusionseinrichtung erfolgt mittels eines Bandabzuges. Die Abzugsgeschwindigkeit des Bandabzugs bestimmt die Geschwindigkeit, mit der das medizinische Instrument durch die Extrusionseinrichtung produziert wird.

Unter einem strukturierten Extrudat kann ein medizinisches Instrument, wie z.B. ein Katheter oder ein Führungsdraht oder ein Halbzeug für ein solches Instrument verstanden werden, oder aber auch langgestreckte Mikrodrähte, -fasern, wie z.B. ummantelte Glasfasern, Drähte oder dergleichen.

Die mit der erfindungsgemäßen Vorrichtung herstellbaren strukturierten Extrudate weisen einen Außendurchmesser von maximal ca. 2,5 mm bzw. 2,3 mm bzw. 2,0 mm bzw. 1,8 mm bzw. 1,6 mm bzw. 1,3 mm bzw. 1 mm auf.

Ein Querschnitt durch medizinische Produkte 30, die die in der Vorteilsbegründung offenbarten stabförmigen Körper mit konturiertem Querschnitt aufweisen, ist in den Figuren 8 bis 15 dargestellt. Diese Figuren zeigen gleichermaßen einen Querschnitt quer zur Produktionsrichtung 3 einer erfindungsgemäßen Pinole 1 im Bereich einer Ausrichtöffnung 8 mit einem zentralen Führungskanal 6 und periphären Führungskanälen 9, sowie ein medizinisches Instrument 30 mit einem zentralen stabförmigen Körper 31 und mehreren peripheren stabförmigen Körpern 32, die zumeist einen konturierten Querschnitt aufweisen.

Im Folgenden werden verschiedene Ausführungsformen eines strukturierten Extrudats bzw. eines medizinischen Instruments 30 mit einem zentralen stabförmigen Körper 31 und mehreren peripheren stabförmigen Körper 32 beschrieben. Der zentrale stabförmige Körper 31 und die mehreren peripheren stabförmigen Körper 32 können dabei konturierte Querschnitt aufweisen, die drei- oder vier- oder vieleckig, trapezförmig oder elliptisch oder oval bzw. bohnenförmig ausgebildet sind.

Gemäß einem ersten Ausführungsbeispiel umfasst ein medizinisches Instrument 30 einen zentralen stabförmigen Körper 31 mit sechseckigem Querschnitt, wobei die Kanten des Sechsecks bezüglich einer quer zu einer Längsrichtung 33 liegenden Ebene konvex ausgebildet sind (Figur 8). Im Bereich der konvexen Kanten des sechseckigen zentralen stabförmigen Körper 31 ist radial umlaufend und gleich beabstandet voneinander jeweils ein elliptischer peripherer stabförmiger Körper 32 derart angeordnet, dass insgesamt in dem strukturierten Extrudat 30 sechs periphere stabförmige Körper 32 angeordnet sind.

Gemäß einer weiteren Ausführungsform des medizinischen Instruments 30 weist der zentrale stabförmige Körper 31 einen sechseckigen Querschnitt mit gleichen Kantenlängen auf, wobei im Bereich der Kanten des Sechsecks des zentralen stabförmigen Körpers 31 radial umlaufend und in etwa gleich beabstandet voneinander sechs periphere stabförmige Körper 32 mit elliptischem Querschnitt vorgesehen sind (Figur 9).

Gemäß einer weiteren Ausführungsform weist der zentrale stabförmige Körper 31 einen runden Querschnitt auf, wobei ebenfalls radial gleich beabstandet voneinander sechs periphere stabförmige Körper 32 mit elliptischem Querschnitt vorgesehen sind (Figur 10).

Gemäß einer weiteren Ausführungsform weist der zentrale stabförmige Körper 31 einen runden Querschnitt auf, wobei die peripheren stabförmigen Körper 32 einen in etwa trapezförmigen Querschnitt aufweisen (Figur 11). Der trapezförmige Querschnitt der peripheren stabförmigen Körper 32 ist dabei derart ausgebildet, dass die in radialer Richtung bezüglich der Längsrichtung 33 verlaufenden Kanten der Trapeze in etwa parallel zueinander angeordnet sind und die beiden in etwa parallel zueinander verlaufenden Kanten des Trapezes bezüglich der Längsrichtung 33 in vertikaler Richtung konkav ausgebildet sind.

Gemäß einer weiteren Ausführungsform des medizinischen Instruments 30 umfasst diese einen zentralen stabförmigen Körper 31 mit fünfeckigem Querschnitt, wobei die Kanten des Fünfecks bezüglich der Längsrichtung 33 konkav ausgebildet sind (Figur 12). Ähnlich dem anhand von Figur 8 beschriebenen Ausführungsbeispiel sind fünf periphere stabförmige Körper 32 vorgesehen, die im Bereich der Kanten des zentralen stabförmigen Körpers 31 angeordnet sind, und wobei eine entlang der Kante des zentralen stabförmigen Körpers 31 ausgebildete Kante der peripheren stabförmigen Körper 32 bezüglich der Längsrichtung 33 konvex ausgebildet ist (Figur 12).

Gemäß einer weiteren Ausführungsform kann der zentrale Führungskanal 6 als gleichseitiges Vieleck, insbesondere als Fünfeck, ausgebildet sein. Demgemäß sind entsprechend fünf periphere Führungskanäle mit elliptischem Querschnitt vorgesehen, deren Kanten bezüglich einer Ebene quer zur Produktionsrichtung 3 konvex ausgebildet sind (Figur 13).

Gemäß einer weiteren Ausführungsform der Pinole 1 kann der zentrale stabförmige Körper 31 rund ausgebildet sein, wobei radial umlaufend und gleich beabstandet voneinander fünf elliptische periphere stabförmige Körper 32 vorgesehen sind, deren Kanten bezüglich einer Ebene quer zur Längsrichtung 33 bezüglich der Längsrichtung 33 konvex ausgebildet ist (Figur 14).

Gemäß einer weiteren Ausführungsform, die im Wesentlichen der anhand von Figur 11 beschriebenen Ausführungsform entspricht, können anstelle von sechs peripheren stabförmigen Körper 32 auch fünf radial gleich beabstandet voneinander angeordnete und in etwa trapezförmig ausgebildete stabförmige Körper 32 vorgesehen sein (Figur 15).

Ein erfindungsgemäßes medizinisches Instrument 30 umfasst in der Regel einen zentralen stabförmigen Körper 31 dessen Querschnitt vorzugsweise rund oder vieleckig ausgebildet ist, wobei die Kanten des Vielecks bezüglich einer Ebene quer zur Längsrichtung 33 konvex oder konkav ausgebildet sein können.

Weiterhin umfasst eine solches medizinisches Instrument 30 vorzugsweise zumindest drei oder vier oder fünf oder sechs oder sieben oder acht oder neun oder mehr periphere stabförmige Körper 32, die elliptisch, vieleckig, trapezförmig ausgebildet sein können und wobei deren Kanten bezüglich der Längsrichtung 33 konkav oder konvex geformt sind.

### Bezugszeichenliste

- 1: Pinole
- 2: zylindrischer Abschnitt
- 3: Produktionsrichtung
- 4: kegelförmiger Abschnitt
- 5: Stufe
- 6: zentraler Führungskanal
- 7: Zuführöffnung
- 8: Ausrichtöffnung
- 9: peripherer Führungskanal
- 10: periphere Ausrichtöffnung
- 11: periphere Zuführöffnung
- 12: Vorrichtung
- 13: Seitenwandung
- 14: Düsenwandung
- 15: Austrittsdüse
- 16: Fixierring
- 17: Hülsenelement
- 18: Durchgangsöffnung
- 19: Stufe
- 20: Extrusionsraum
- 21: Kegelstumpf
- 22: Schmelzekanal
- 23: Anordnungswandung
- 24: Eintrittsöffnung
- 25: Austrittsöffnung
- 26: Kegelstumpf
- 27: Rinne/Ausbuchtung
- 28: Einbuchtung
- 29: stabförmiger Körper
- 30: strukturiertes Extrudat/medizinisches Instrument
- 31: zentraler stabförmiger Körper
- 32: peripherer stabförmigen Körper
- 33: Längsrichtung des medizinischen Instruments bzw. stabförmigen Körpers

## Patentansprüche

1. Vorrichtung zum Extrudieren eines strukturierten Extrudats, umfassend ein Gehäuse, wobei das Gehäuse eine umlaufende Seitenwandung aufweist, die an einem in Produktionsrichtung vorne liegenden Ende mit einer eine Austrittsdüse aufweisenden Düsenwandung und am entgegen der Produktionsrichtung hinten liegenden Ende mit einer Pinole versehen ist, wobei der Raum im Gehäuse zwischen der Pinole, der Seitenwandung und der Austrittsdüse einen Extrusionsraum begrenzt und das Gehäuse im Bereich des Extrusionsraumes mit einer Polymerzuführeinrichtung verbindbar ist,
dass in der Pinole zumindest ein sich in Produktionsrichtung erstreckender Führungskanal ausgebildet ist, um zumindest einen stabförmigen Körper von einer Einrichtung zum Zuführen stabförmiger Körper bis in den Extrusionsraum einzubringen, und
der zumindest eine Führungskanal in etwa in geradliniger Flucht zur Austrittsdüse angeordnet ist, wobei der Führungskanal über seine gesamte Länge eine im wesentlichen konstante Konizität aufweist, wobei sich der Führungskanal über die gesamte Länge der Pinole erstreckt **dadurch gekennzeichnet,**
**dass** zumindest ein peripherer Führungskanal neben dem zentralen Führungskanal vorgesehen ist, der
über seine gesamte Länge eine im Wesentlichen konstante Konizität aufweist.

2. Vorrichtung gemäß Anspruch 1,
**dadurch gekennzeichnet,**
**dass** der zentrale Führungskanal und/oder der periphere Führungskanal einen konturierten Querschnitt
aufweisen, der elliptisch oder oval bzw. bohnenförmig oder trapezförmig oder drei- oder vier- oder vieleckig ausgebildet ist.

3. Vorrichtung gemäß Anspruch 1 oder 2,
**dadurch gekennzeichnet,**
**dass** der periphere Führungskanal gegenüber der Produktionsrichtung in einem Winkel von 0° bis 30° bzw. von 2,5° bis 15° und insbesondere von 5° bis 10° geneigt ist.

4. Vorrichtung gemäß einem der Ansprüche 1 bis 3,
**dadurch gekennzeichnet,**
**dass** zumindest drei den zentralen Durchgangskanal konzentrisch umgebende periphere Führungskanäle vorgesehen sind

5. Vorrichtung gemäß einem der Ansprüche 1 bis 4,
**dadurch gekennzeichnet,**
**dass** eine Innenfläche/Wandung eines Führungskanals eine Oberflächenrauheit R_{A} ≤1,0 µm aufweist

6. Vorrichtung gemäß einem der Ansprüche 1 bis 5,
**dadurch gekennzeichnet,**
**dass** eine in Produktionsrichtung vorne liegende Öffnung des zentralen Führungskanals einen Durchmesser von 0,2 mm bis 0,4 mm und insbesondere von 0,3 mm und eine in Produktionsrichtung hinten liegende Öffnung des zentralen Führungskanals einen Durchmesser von 2,0 mm bis 4,0 mm und insbesondere von 3,0 mm aufweist, und/oder dass eine in Produktionsrichtung vorne liegende Öffnung des peripheren Führungskanals einen Durchmesser von 0,1 mm bis 0,3 mm und insbesondere von 0,2 mm und eine in Produktionsrichtung hinten liegende Öffnung des peripheren Führungskanals einen Durchmesser von 3,0 mm bis 5,0 mm und insbesondere von 4,0 mm aufweist.

7. Vorrichtung nach einem der Ansprüche 1 bis 6,
**dadurch gekennzeichnet,**
**dass** die Düsenwandung und die Pinole im Bereich des Extrusionsraumes als ein sich in Produktionsrichtung konisch verjüngender Kegelstumpf ausgebildet sind, wobei der Kegelstumpf der Pinole eine bezüglich des Extrusionsraumes konkav ausgebildete Mantelwandung und die Düsenwandung eine bezüglich des Extrusionsraumes konvex ausgebildete Mantelwandung aufweist und/oder dass in Produktionsrichtung nach der Austrittsdüse eine Kühleinrichtung, insbesondere ein Wasserbad zum Kühlen der Führungsdrähte oder der Katheter angeordnet ist und/oder dass in Produktionsrichtung nach der Kühleinrichtung eine Abzugseinrichtung angeordnet ist, die ausgebildet ist, um ein strukturiertes Extrudat unter Spannung zu halten und/oder dass in Produktionsrichtung nach der Kühleinrichtung eine Ausrichteinrichtung und/oder dass in Produktionsrichtung vor dem Gehäuse eine Einrichtung zum Zuführen stabförmiger Körper angeordnet ist.

8. Vorrichtung nach einem der Ansprüche 1 bis 7,
**dadurch gekennzeichnet,**
**dass** die Austrittsdüse einen Schmelzekanal aufweist, wobei der Schmelzekanal sich in Produktionsrichtung konisch verjüngend als ein ringförmiger Kegelstumpf mit einer Anordnungswandung ausgebildet ist, die im Querschnitt insbesondere gewellt oder gezackt mit Ein- und Ausbuchtungen ausgebildet sind, wobei diese Konturen jeweils als Rinnen bezeichnet werden.

9. Vorrichtung gemäß Anspruch 8,
**dadurch gekennzeichnet,**
**dass** diese Rinnen an einem in Produktionsrichtung liegenden Ende der Austrittsdüse in einen kreisförmigen Querschnitt übergehen und/oder
**dass** die Anzahl der konturierten Rinnen der Anzahl der peripheren Führungskanäle 9 entspricht, wobei vorzugsweise die Einbuchtungen axial fluchtend zu den Führungskanälen angeordnet sind.

10. Verfahren zum Extrudieren eines strukturierten Extrudats, bei dem zumindest zwei stabförmige Körper über eine Einrichtung zum Zuführen stabförmiger Körper einer Extrusionseinrichtung in einer Produktionsrichtung zugeführt und in der Extrusionseinrichtung mit einem Polymer umhüllt werden und
beim Austritt aus einer Austrittsdüse der Extrusionseinrichtung in Produktionsrichtung ihre endgültige Form erhalten,
wobei ein stabförmiger Körper mittels eines in einer Pinole ausgebildeten und sich in Produktionsrichtung erstreckenden zentralen Führungskanals mit im wesentlichen konstanter Konizität
der Extrusionseinrichtung bis in den Bereich in Produktionsrichtung vor der Austrittsdüse in einen Extrusionsraum geführt und ausgerichtet wird,
**dadurch gekennzeichnet,**
**dass** der andere stabförmige Körper mittels zumindest eines in der Pinole ausgebildeten und sich in Produktionsrichtung erstreckenden peripheren Führungskanals in der
Extrusionseinrichtung bis in den Bereich in Produktionsrichtung vor der Austrittsdüse in den Extrusionsraum geführt und ausgerichtet wird, wobei der der periphere Führungskanal neben dem Führungskanal vorgesehen ist, und wobei der periphere Führungskanal über seine gesamte Länge eine im Wesentlichen konstante Konizität aufweist.

11. Verfahren nach Anspruch 10,
**dadurch gekennzeichnet,**
**dass** beim Zuführen zumindest zweier stabförmiger Körper die Zwischenräume zwischen den stabförmigen Körpern mit Polymer beaufschlagt werden, wodurch diese in das Polymer eingebettet werden und/oder
**dass** zumindest ein stabförmiger Körper in einem peripheren Führungskanal der Pinole der Extrusionseinrichtung in einem Winkel von 0° bis 30° bzw. von 2,5° bis 15° und insbesondere von 5° bis 10° geneigt geführt wird und/oder
**dass** die Zuführung über den zentralen Führungskanal und/oder den peripheren Führungskanal erfolgt, wobei zumindest einer dieser Führungskanäle einen konturierten Querschnitt aufweist, der elliptisch oder oval bzw. bohnenförmig oder trapezförmig oder drei- oder vier- oder vieleckig ausgebildet ist.

12. Verfahren nach einem der Ansprüche 10 oder 11,
**dadurch gekennzeichnet,**
**dass** eine Vorrichtung gemäß einem der Ansprüche 1 bis 6 verwendet wird.

## Claims

1. Device for extruding a structured extrudate, comprising:
a housing, wherein the housing has a circumferential lateral wall which, at a front end in the direction of production, is provided with a nozzle wall with an outlet nozzle, and at the back end contrary to the direction of production, is provided with a global sleeve, wherein the space in the housing between the global sleeve, the lateral wall and the outlet nozzle delimits an extrusion space, and the housing in the region of the extrusion space can be connected to a polymer feeding appliance,
wherein at least one guide channel extending in the direction of production is formed in the global sleeve, in order to introduce at least one rod-shaped body from a feeding appliance for rod-shaped bodies into the extrusion space, and
the at least one guide channel is arranged roughly in straight alignment relative to the outlet nozzle, wherein the guide channel has a substantially constant conicity over its entire length, wherein the guide channel extends along the entire length of the global sleeve,
**characterized in that**
at least one peripheral guide channel is provided besides the central guide channel, which has substantially constant conicity over its entire length.

2. Device according to Claim 1,
**characterized in that**
the central guide channel and/or the peripheral guide channel have a contoured cross section which is elliptic or oval or else bean-shaped or trapezoidal or three- or four- or multi-cornered.

3. Device according to Claim 1 or 2,
**characterized in that**
the peripheral guide channel is inclined relative to the direction of production at an angle from 0° to 30° or from 2.5° to 15° and, in particular, from 5° to 10°.

4. Device according to one of Claims 1 to 3,
**characterized in that**
at least three peripheral guide channels are provided concentrically surrounding the central guide channel.

5. Device according to one of Claims 1 to 4,
**characterized in that**
an inner surface/wall of a guide channel has a surface roughness R_{A} ≤ 1.0 µm.

6. Device according to one of claims 1 to 5,
**characterized in that**
an opening of the central guide channel at the front end in the direction of production has a diameter of 0.2 mm to 0.4 mm and, in particular, of 0.3 mm, and an opening of the central guide channel at the back end in the direction of production has a diameter of 2.0 mm to 4.0 mm and, in particular, of 3.0 mm, and/or
an opening of the peripheral guide channel at the front end in direction of production has a diameter of 0.1 mm to 0.3 mm and, in particular, of 0.2 mm, and an opening of a peripheral guide channel at the back end in the direction of production has a diameter of 3.0 mm to 5.0 mm and, in particular, of 4.0 mm.

7. Device according to one of Claims 1 to 6,
**characterized in that**
the nozzle wall and the global sleeve in the region of the extrusion space are formed as a conically tapered frustum in the direction of production, wherein the frustum of the global sleeve comprises a jacket wall which is concave relative to the extrusion space and the nozzle wall comprises a jacket wall which is convex relative to the extrusion space, and/or in the direction of production behind the outlet nozzle, a cooling unit, in particular a water bath for cooling the guide wires or catheters, is arranged, and/or in the direction of production behind the cooling unit, a take-off unit is arranged, which is designed to hold a structured extrudate under tension, and/or in the direction of production behind the cooling unit, an alignment unit is arranged, and/or in the direction of production in front of the housing, an appliance for feeding rod-shaped bodies is arranged.

8. Device according to one of Claims 1 to 7,
**characterized in that**
the outlet nozzle provides a melt channel, wherein the melt channel is designed as a ring-shaped frustum with an assembly wall which tapers conically in the direction of production, which assembly wall has a particularly wavy or jagged design in cross section with indentations and protrusions, wherein these contours are each referred to as grooves.

9. Device according to Claim 8,
**characterized in that**
these grooves transition into a circular cross section at one end of the outlet nozzle lying in the direction of production, and/or
the number of contoured grooves corresponds to the number of peripheral guide channels 9, wherein the indentations are preferably arranged in axial alignment with the guide channels.

10. Method for extruding a structured extrudate, wherein
at least two rod-shaped bodies are fed via an appliance for feeding rod-shaped bodies from an extrusion device in a direction of production and encased with a polymer in the extrusion device, and,
upon release from an outlet nozzle of the extrusion device in the direction of production, obtain their final shape,
wherein a rod-shaped body is guided and aligned by means of a central guide channel formed in the global sleeve and extending in the direction of production with substantially constant conicity of the extrusion device up to the region in the direction of production in front of the outlet nozzle into an extrusion space,
**characterized in that**
the other rod-shaped body is guided and aligned by means of at least one peripheral guide channel formed in the global sleeve and extending in the direction of production in the extrusion device up to the region in the direction of production in front of the outlet nozzle into the extrusion space, wherein the peripheral channel is provided alongside the guide channel, and wherein the peripheral guide channel has substantially constant conicity over its entire length.

11. Method according to Claim 10,
**characterized in that**
during the feeding of at least two rod-shaped bodies, the gaps between the rod-shaped bodies have polymer applied to them, as a result of which they become embedded in the polymer and/or at least one rod-shaped body is inserted into a peripheral guide channel of the global sleeve of the extrusion device at an angle from 0° to 30° or from 2.5° to 15° and, in particular, from 5° to 10°, and/or
feeding occurs via the central guide channel and/or the peripheral guide channel, wherein at least one of these guide channels has a contoured cross section which is elliptic or oval or else bean-shaped or trapezoidal or three- or four- or multi-cornered.

12. Method according to one of Claims 10 or 11,
**characterized in that**
a device according to one of Claims 1 to 6 is used.

## Revendications

1. Dispositif d'extrusion d'un extrudat structuré, comprenant
un boîtier, dans lequel le boîtier présente une paroi latérale circonférentielle qui est pourvue d'une paroi de buse présentant une buse de sortie à l'extrémité avant dans la direction de production et d'un fourreau à l'extrémité arrière opposée à la direction de production, dans lequel l'espace dans le boîtier entre le fourreau, la paroi latérale et la buse de sortie délimite un espace d'extrusion et le boîtier peut être relié à un dispositif d'alimentation en polymère au niveau de l'espace d'extrusion,
qu'au moins un canal de guidage s'étendant dans la direction de production est formé dans le fourreau, afin d'introduire au moins un corps en forme de tige d'un dispositif d'introduction de corps en forme de tige dans l'espace d'extrusion, et
l'au moins un canal de guidage est disposé approximativement en ligne droite avec la buse de sortie, dans lequel le canal de guidage présente une conicité sensiblement constante sur toute sa longueur, dans lequel le canal de guidage s'étend sur toute la longueur du fourreau, **caractérisé en ce que** :
au moins un canal de guidage périphérique est prévu à côté du canal de guidage central, qui présente une conicité sensiblement constante sur toute sa longueur.

2. Dispositif selon la revendication 1,
**caractérisé en ce que,**
le canal de guidage central et/ou le canal de guidage périphérique présentent une section transversale profilée, qui a une configuration elliptique ou ovale, respectivement en forme de haricot ou trapézoïdale ou triangulaire, carrée ou polygonale.

3. Dispositif selon la revendication 1 ou 2,
**caractérisé en ce que,**
le canal de guidage périphérique est incliné par rapport à la direction de production d'un angle de 0° à 30° ou de 2,5° à 15° et notamment de 5° à 10°.

4. Dispositif selon une des revendications 1 à 3,
**caractérisé en ce que,**
au moins trois canaux de guidage périphériques entourant concentriquement le canal de passage central sont prévus.

5. Dispositif selon une des revendications 1 à 4,
**caractérisé en ce que,**
une surface intérieure /paroi d'un canal de guidage présente une rugosité de surface R_{A} ≤ 1,0 µm.

6. Dispositif selon une des revendications 1 à 5,
**caractérisé en ce que,**
une ouverture du canal de guidage central située à l'avant dans la direction de production présente un diamètre de 0,2 mm à 0,4 mm et notamment de 0,3 mm et une ouverture du canal de guidage central située à l'arrière dans la direction de production présente un diamètre de 2,0 mm à 4,0 mm et notamment de 3,0 mm, et/ou
qu'une ouverture du canal de guidage périphérique située à l'avant dans la direction de production présente un diamètre de 0,1 mm à 0,3 mm et notamment 0,2 mm et une ouverture du canal de guidage périphérique située à l'arrière dans la direction de production présente un diamètre de 3,0 mm à 5,0 mm et notamment de 4,0 mm.

7. Dispositif selon une des revendications 1 à 6,
**caractérisé en ce que,**
la paroi de la buse et le fourreau au niveau de l'espace d'extrusion sont conçus comme un cône tronqué qui se rétrécit coniquement dans la direction de production, dans lequel le cône tronqué du fourreau présente une paroi enveloppante à configuration concave par rapport à l'espace d'extrusion et la paroi de la buse présente une paroi enveloppante à configuration convexe par rapport à l'espace d'extrusion et/ou que dans la direction de production après la buse de sortie un dispositif de refroidissement, notamment un bain d'eau pour refroidir les fils de guidage ou les cathéters, est disposé et/ou que dans la direction de production après le dispositif de refroidissement est disposé un dispositif d'extraction, qui est configuré pour maintenir un extrudat structuré sous tension et/ou que dans la direction de production, après le dispositif de refroidissement, un dispositif d'alignement et/ou que, dans la direction de production, avant le boîtier, un dispositif d'introduction de corps en forme de tige est disposé.

8. Dispositif selon une des revendications 1 à 7,
**caractérisé en ce que,**
la buse de sortie présente un canal de fusion, dans lequel le canal de fusion se rétrécit coniquement dans la direction de production sous la forme d'un cône tronqué annulaire comportant une paroi d'agencement, qui, en section transversale, ont une configuration notamment ondulée ou dentelée avec des indentations et des renflements, dans lequel ces profils sont respectivement appelés rainures.

9. Dispositif selon la revendication 8,
**caractérisé en ce que,**
ces rainures se rejoignent en une section transversale circulaire à une extrémité de la buse de sortie située dans la direction de production et/ou
que le nombre de rainures profilées correspond au nombre de canaux de guidage périphériques 9, dans lequel les indentations sont de préférence disposées axialement en alignement avec les canaux de guidage.

10. Procédé d'extrusion d'un extrudat structuré, dans lequel
au moins deux corps en forme de tige sont introduits via un dispositif d'introduction es corps en forme de tige dans un dispositif d'extrusion dans une direction de production et enrobés d'un polymère dans le dispositif d'extrusion, et
reçoivent leur forme définitive à la sortie d'une buse de sortie du dispositif d'extrusion dans la direction de production,
dans lequel
un corps en forme de tige est guidé et aligné au moyen d'au moins un canal de guidage central s'étendant dans la direction de production et réalisé dans un fourreau avec une conicité sensiblement constante dans le dispositif d'extrusion jusqu'à une chambre d'extrusion située avant la buse de sortie dans la direction de production,
**caractérisé en ce que,**
l'autre corps en forme de tige est guidé et aligné au moyen d'au moins un canal de guidage périphérique s'étendant dans la direction de production et réalisé dans le fourreau dans le dispositif d'extrusion jusqu'à la chambre d'extrusion située avant la buse de sortie dans la direction de production,
dans lequel le canal de guidage périphérique est prévu à côté du canal de guidage, et dans lequel le canal de guidage périphérique présente une conicité sensiblement constante sur toute sa longueur.

11. Procédé selon la revendication 10,
**caractérisé en ce que,**
lorsqu'au moins deux corps en forme de tige sont introduits, les espaces intermédiaires entre les corps en forme de tige sont recouverts avec du polymère, de sorte qu'ils soient noyés dans le polymère et/ou
qu'au moins un corps en forme de tige est guidé incliné dans un canal de guidage périphérique du fourreau du dispositif d'extrusion selon un angle de 0° à 30° ou de 2,5° à 15° et notamment de 5° à 10° et/ ou
que l'introduction s'effectue par le canal de guidage central et/ou le canal de guidage périphérique, dans lequel au moins un de ces canaux de guidage présente une section transversale profilée, qui a une configuration elliptique ou ovale, respectivement en forme de haricot ou trapézoïdale ou triangulaire, carrée ou polygonale.

12. Procédé selon une des revendications 10 ou 11,
**caractérisé en ce que**,
un dispositif selon une des revendications 1 à 6 est utilisé.
